# EUROPEAN PATENT SPECIFICATION

(11) **EP 1 309 347 B1**
(45) Date of publication and mention of the grant of the patent: **27.02.2013**
(21) Application number: 01979228.2
(22) Date of filing: 10.08.2001
(51) Int. Cl.: A61K 39/395

(54) **METHOD AND COMPOSITION FOR ALTERING A B CELL MEDIATED PATHOLOGY**
VERFAHREN UND ZUSAMMENSETZUNG ZUR ÄNDERUNG EINER B ZELL-MEDIIERTEN PATHOLOGIE
METHODE ET COMPOSITION DE MODIFICATION D'UNE PATHOLOGIE A MEDIATION ASSUREE PAR LES LYMPHOCYTES B

(30) Priority: 11.08.2000 US 224723 P; 11.08.2000 US 224722 P; 23.03.2001 US 279079 P
(43) Date of publication of application: 14.05.2003
(73) Proprietor: MMRGLOBAL, INC., Los Angeles CA 90010 (US)
(72) Inventor: GOLD, Daniel, P., Del Mar, CA 92014 (US); SHOPES, Robert, J., San Diego, CA 92102 (US)
(74) Representative: Campbell, Patrick John Henry
(86) International application number: PCT/US2001/025204
(87) International publication number: WO 2002/013862

(56) References cited:
- WO-A1-97/41244
- US-A- 4 816 249
- US-A- 5 972 334
- TAO M-H ET AL: "IDIOTYPE/GRANULOCYTE-MACROPHAGE COLONY-STIMULATING FACTOR FUSION PROTEIN AS A VACCINE FOR B-CELL LYMPHOMA" NATURE, MACMILLAN JOURNALS LTD. LONDON, GB, vol. 362, 22 April 1993 (1993-04-22), pages 755-758, XP002031411 ISSN: 0028-0836
- SELMAYR M ET AL: "B-CELL LYMPHOMA IDIOTYPES CHIMERIZED BY GENE TARGETING CAN INDUCE TUMOR IMMUNITY" CANCER GENE THERAPY, NORWALK, CT, US, vol. 7, no. 3, March 2000 (2000-03), pages 501-506, XP000972429 ISSN: 0929-1903
- BENVENUTI F ET AL: "Anti-idiotypic DNA vaccines for lymphoma immunotherapy require the presence of both variable region genes for tumor protection." GENE THERAPY, vol. 7, no. 7, April 2000 (2000-04), pages 605-611, XP001098032 ISSN: 0969-7128
- CASPAR CLEMENS B ET AL: "Idiotype vaccines for non-Hodgkin's lymphoma induce polyclonal immune responses that cover mutated tumor idiotypes: Comparison of different vaccine formulations." BLOOD, vol. 90, no. 9, 1997, pages 3699-3706, XP002211227 ISSN: 0006-4971 cited in the application
- VEELKEN H ET AL: "RAPID EXPRESSION CLONING OF B CELL LYMPHOMA-DERIVED IMMUNOGLOBULIN GENES FOR ANTI-IDIOTYPIC VACCINATION AND FUNCTIONAL ANALYSES OF FAB-FRAGMENTS" BLOOD, W.B. SAUNDERS, PHILADELPHIA, VA, US, vol. 90, no. 10 SUPPL 1 PAR, 15 November 1997 (1997-11-15), page 515A XP000992835 ISSN: 0006-4971
- ALTMANN F ET AL: "Insect cells as hosts for the expression of recombinant glycoproteins.", GLYCOCONJUGATE JOURNAL FEB 1999 LNKD- PUBMED:10612411, vol. 16, no. 2, February 1999 (1999-02), pages 109-123, ISSN: 0282-0080
- FREEDMAN ARNOLD ET AL: "Placebo-controlled phase III trial of patient-specific immunotherapy with mitumprotimut-T and granulocyte-macrophage colony-stimulating factor after rituximab in patients with follicular lymphoma", JOURNAL OF CLINICAL ONCOLOGY, AMERICAN SOCIETY OF CLINICAL ONCOLOGY, US, vol. 27, no. 18, 20 June 2009 (2009-06-20) , pages 3036-3043, XP009142239, ISSN: 0732-183X [retrieved on 2009-05-04]
- ZU PUTLITZ J ET AL: "Antibody production in baculovirus-infected insect cells.", BIO/TECHNOLOGY (NATURE PUBLISHING COMPANY) JUL 1990 LNKD- PUBMED:1367456, vol. 8, no. 7, July 1990 (1990-07), pages 651-654, XP009142302, ISSN: 0733-222X
- MALONEY ET AL, [Online] FCgammaRIIIA receptor polymorphism do not influence the Outcome of treatment with Rituximab followed by active immunotherapy with Mitumprotimut-T (Specifid, Id-KLH,FavId) Retrieved from the Internet: <URL:tier1group.com/downloads/media/c3/PDF> [retrieved on 2010-12-09]
- KWAK L W ET AL: "Vaccination with syngeneic, lymphoma-derived immunoglobulin idiotype combined with granulocyte/macrophage colony-stimulating factor primes mice for a protective T-cell response.", PROCEEDINGS OF THE NATIONAL ACADEMY OF SCIENCES OF THE UNITED STATES OF AMERICA 1 OCT 1996 LNKD- PUBMED:8855293, vol. 93, no. 20, 1 October 1996 (1996-10-01), pages 10972-10977, ISSN: 0027-8424
- POTTER K N ET AL: "ANTIBODY PRODUCTION IN THE BACULOVIRUS EXPRESSION SYSTEM", INTERNATIONAL REVIEWS OF IMMUNOLOGY, HARWOOD ACADEMIC PUBLISHERS, LONDON, GB, vol. 10, no. 2/03, 1 January 1993 (1993-01-01), pages 103-112, XP000770700, ISSN: 0883-0185
- TAN W ET AL: "Expression and purification of a secreted functional mouse/human chimaeric antibody against bacterial endotoxin in baculovirus-infected insect cells.", BIOTECHNOLOGY AND APPLIED BIOCHEMISTRY AUG 1999 LNKD- PUBMED:10467120, vol. 30 ( Pt 1), August 1999 (1999-08), pages 59-64, ISSN: 0885-4513
- MROCZKOWSKI B S ET AL: "Secretion of thermostable DNA polymerase using a novel baculovirus vector.", THE JOURNAL OF BIOLOGICAL CHEMISTRY 6 MAY 1994 LNKD- PUBMED:8175786, vol. 269, no. 18, 6 May 1994 (1994-05-06), pages 13522-13528, ISSN: 0021-9258

## Description

### FIELD OF THE INVENTION

This invention relates generally to the field of immunology and immunotherapy. More specifically, this invention relates to compositions for therapy of B cell lymphomas.

### BACKGROUND OF THE INVENTION

The immune system produces both antibody-mediated and cell-mediated responses. Each type of immune response is regulated by a type of lymphocyte, B cells (for antibody-mediated response) and T cells (for cell-mediated response). B cells initially recognize an antigen when the antigen binds to the IgM and IgD molecules on the B cell's surface. Each B cell clone recognizes only specific antigens due to the unique idiotype of that clone. Upon recognition of the antigen, B cells internalize and process the antigen for presentation via MHC class II molecules. B cells can thereby function as an antigen presenting cell ("APC") for T cells. T cells bind to portions of foreign proteins (antigens) when portions of the protein associate with a major histocompatibility complex molecule ("MHC"), typically on an APC, in which the antigen is digested into fragments and presented on the surface of the APC bound to its MHC.

Several types of cancers have their origin in the circulatory system. Among the major types are: leukemias, a neoplasm of the bone marrow and blood; myelomas, a cancer of B cells; and lymphomas, a group of cancers that originate in the lymphatic system. Lymphomas can be further classified into several groups; one of these groups is the non-Hodgkin's lymphomas which, in turn, forms a diverse group of cancers. Three broad categories of these lymphomas are defined according to the International Working Formulation for tumor classification, low grade, intermediate grade and high grade, which differ in their curability and aggressiveness (Cheson, et al., "Report of an International Workshop to Standardize Response Criteria for Non-Hodgkin's Lymphomas," J. Clin Oncol. 17(4):1244,1999). Overall, these lymphomas collectively rank fifth in the United States in terms of cancer incidence and mortality, and approximately 50,000 new cases are diagnosed each year.

In a recent study which examined fifty-one case isolates of high-grade non-Hodgkins's lymphoma (NHL), forty-three were shown to be derived from B cells while eight were shown to be derived from T cells (Brown et al., Histopathology 14:621-27, 1989). Therefore, treatments directed specifically towards pathological B cells would be valuable in the treatment of non-Hodgkin's lymphomas and myelomas.

Initial attempts in the field to develop an immunology-based treatment directed at antigens uniquely produced by malignant B cells involved laboriously isolating and purifying idiotypic (Id) proteins directly from the pathological B cells. This purified protein was first used in model systems to treat the associated lymphoma. It was demonstrated that this active immunization against idiotypic determinants on isolated proteins could produce resistance to tumor growth in a mouse model system (Daley et al., J.Immunol. 120(5):1620-24, 1978; Sakato et al., Microbiol. Immunol. 23(9):927-31, 1979). This phenomenon of resistance to tumor growth has been subsequently reproduced in a number of additional experimental tumor models (Stevenson et al., J.Immunol. 130(2):970-03, 1983; George et al., J.Immunol. 141(6):2168-74, 1988; Kwak, et al., Blood 76(11):2411-17,1990).

Among the first attempts at bringing this idea and technology into the clinic was very labor intensive and utilized mouse monoclonal antibodies generated against proteins isolated from the patients' individual lymphomas following biopsy. Meeker and coworkers generated mouse monoclonal anti-idiotype antibodies for treatment of eleven patients after most had already undergone conventional lymphoma therapy (Meeker et al., Blood 65:1349-63, 1985). Positive results were obtained in roughly half the patients, with one case of apparent remission. In some of the patients, however, the lymphoma cells developed a resistance to the antibody via switching the class of cell surface-expressed antibodies (Meeker et al., N Engl J Med. 312:1658-65, 1985).

Another way a B cell lymphoma clone developed resistance to anti-idiotypic antibodies is via a somatic mutation in the CDR2 region (Cleary et al., Cell 44:97-106, 1986), thereby evading recognition. While this passive immunity approach for treatment has the advantage that it only requires isolation and purification of a relatively minor amount of idiotypic protein from a patient for raising an immune response in a mouse, the usefulness for treating lymphomas with monoclonal antibodies directed at idiotypes is limited. In the absence of a robust and convenient way to produce large quantities of idiotypic protein, however, this could prove to be the only practical way to exploit the abilities of the immune system to directly attack the idiotype of a B cell lymphoma.

Kwak *et al.* pursued a different approach and attempted the active immunization of patients using proteins purified from their own unique lymphomas in spite of the logistical requirement for isolating large quantities of idiotypic proteins (Kwak et al., N. Engl. J. Med. 327:1209-15, 1992). Patients who had minimal or no disease following chemotherapy were treated by vaccination with autologous idiotype proteins. In order to obtain sufficient quantities of idiotypic proteins for vaccination, lymphoma cells obtained by biopsy were fused with an established cell line to facilitate their growth in tissue culture, and the secreted idiotype proteins were purified via chromatography. Large scale application of this method of immunization is precluded due to the extreme labor requirements, technical barriers, and prohibitive costs. Additionally, concerns have recently been raised concerning the viral loads associated with protein production in mammalian cells.

In a following paper, Hsu *et al.* reported on the phase I/II of the above clinical trial utilizing vaccination of the idiotype conjugated to keyhole limpet hemocyanin (KLH) in the treatment of B-cell lymphoma (Hsu et al., Blood 89:3129-35,1997). After standard chemotherapy, 41 patients with refractory non-Hodgkin's B-cell lymphoma were vaccinated with a tumor-specific idiotype. As per Kwak *et al* (1992), *supra,* the tumor-specific idiotype antigens were obtained by chromatographic purification of proteins produced by the patients' hybridomas. These proteins were therefore composed of the entire variable and constant regions of the patient's own immunoglobulin from the patients' lymphomas. The results showed that the generation of an anti-idiotype response correlated with improved clinical outcome. The duration of freedom from disease progression and overall survival of all patients mounting an anti-idiotype cellular immune response were significantly prolonged compared to those patients who did not mount an immune response. This study confirms that patients with B-cell lymphomas can be induced to make a specific immune response against tumor idiotype (Id) protein. Furthermore, the ability to generate an anti-idiotype immune response correlates with a more favorable clinical outcome. However, to treat each individual patient, lymphoma cells obtained by biopsy must be fused to established cell lines in order to allow the production of sufficient protein to vaccinate a typical patient. This process would be difficult or impractical to use on a commercial scale.

More recently, Bendandi *et al.* demonstrated idiotypic, patient-specific vaccination-induced remissions in patients with follicular lymphoma (Bendandi et al., Nat. Med. 5:1171-77, 1999). Following standard chemotherapy, twenty patients demonstrating complete clinical remission were vaccinated using patient-specific idiotypic proteins accompanied by granulocyte-monocyte colony-stimulating factor (GM-CSF; *see infra.*). Molecular analysis of the translocations characteristic of this lymphoma was conducted prior to chemotherapy, at clinical remission and following vaccination therapy. Eight of eleven patients with detectable translocations after chemotherapy-induced remission were found to undergo complete molecular remission following this vaccination. Tumor-specific cytotoxic CD8⁺ and CD4⁺ T cells were found in 19 of 20 patients. Tumor-specific antibodies were also detected but were not found to be required for remission. Again, this study used idiotypic proteins made up of the entire variable and constant region of the immunoglobulin found associated with the patient's lymphoma and produced by heterohybridoma fusion.

Therefore, directing an immune response to the idiotype of cells is a promising approach, but the above techniques are limited by the requirement of producing sufficient quantities of idiotypic proteins from each patient's lymphoma cells.

The concept of anti-idiotypic immunity against B cell tumors has also been used in the case of multiple myeloma. Results have been reported by Kwak and coworkers regarding its use in enhancing the specific efficacy of allogeneic marrow grafts by pre-immunizing the donor with myeloma IgG isolated from the patient (Kwak et al., Lancet 345 (8956):1016-20, 1995). Also, Massaia and coworkers vaccinated patients in remission following high-dose chemotherapy, followed by peripheral blood stem cell transplantation (Massaia et al., Blood 94:673-83, 1999).

Granulocyte-monocyte colony-stimulating factor (GM-CSF), used above in Bendandi *et al.'s* study, is a hematopoietic growth factor which stimulates proliferation and differentiation of hematopoietic progenitor cells. This cytokine also plays a role in shaping cellular immunity by augmenting T-cell proliferation (Santoli et al., J.Immunol. 141 (2):519-26,1988). increasing expression of adhesion molecules on granulocytes and monocytes (Young et al., J.Immunol. 145(2):607-15, 1990; Grabstein et al., Science 232(4749):506-08, 1986), and augmenting antigen presentation (Morrissey et al., J.Immunol. 139(4):1113-9, 1987; Heufler et al., J. Exp. Med. 167(2):700-05, 1988; Smith et al., J.Immunol. 144(5):1777-82, 1990).

Cell-based vaccines genetically engineered to produce GM-CSF have been shown to induce cellular immune responses capable of eliminating systemic lymphomas in preclinical models. This effect is mediated exclusively through activation of the cellular arm of the immune system (Levitsky et al., J. Immuno. 156(10): 3858-65, 1996). Similarly, low doses of free GM-CSF have been shown to enhance the protective anti-tumor immunity induced by idiotype protein-KLH immunization because of its ability to enhance immunity through an effect on the CD8 cells (Kwak et al., Proc. Natl. Acad. Sci. USA 93(20):10972-77, 1996. In one study, GM-CSF was shown to be the best immunomodulator to generate anti-tumor immunity among those tested in a model system (Dranoff, G., Proc. Natl. Acad. Sci. USA 90(8):3539-43, 1993.)

GM-CSF has also been used as a portion of a chimeric protein used to generate an immune response in model systems. Chen and Levy (Chen and Levy, J. Immunol. 154(7):3105-17, 1995; U. S. Patent No. 6,099,846) studied the production of mouse monoclonal antibodies using a chimeric protein containing a portion of GM-CSF plus a portion of an antigen of interest, namely an idiotypic region obtained from a murine B-cell tumor, 38C13, both fused to portions of human immunoglobulin chains. Chen and coworkers have also studied fusion proteins where the GM-CSF moiety has been replaced by portions of IL-2 or IL-4 (Chen et al., J.Immunol. 153(10):4775-87, 1994). One explanation for the requirement of including the GM-CSF moiety (or interleukin moiety) was to augment the effect of low levels of chimeric protein produced by the mammalian cell expression system. However, the use of purified GM-CSF co-administered with a chimeric protein to enhance the immune response of a vaccination has not been demonstrated.

With the advent of recombinant DNA technology, heavy and light chain cDNA molecules can now be cloned from hybridomas or from combinatorial libraries employing the polymerase chain reaction (PCR). This recombinant DNA technology allows researchers to manipulate the effector function or the binding function of a selected monoclonal antibody. In addition, combinatorial libraries of immunoglobulins can be generated by cloning a large number of V_{L} and V_{H} genes, randomly assorting them to create a library of different binding specificities, expressing them in *E. coli,* then screening the stochastic library for clones with the desired binding affinities (Huse et al., Science 246(4935):1275-81, 1989). Using this recombinant approach, human antibodies were cloned with high affinity and specificity for tetanus toxoid from a randomized combinatorial library expressed in *E*. *coli* (Mullinax et al., Proc. Natl. Acad. Sci. 87(20):8095-99,1990). The immunoglobulin genes were cloned from activated B-cells into bacteriophage vectors using the polymerase chain reaction (PCR) with specific primers. The H and L chains were randomly combined and co-expressed in *E. coli* to comprise a library of 10⁷ members. This combinatorial library was screened with ¹²⁵I-tetanus toxoid and 0.2% of the clones displayed binding activity (Mullinax *et al., supra*). In addition, murine monoclonal antibodies have also been identified using a similar approach (Huse *et al., supra*; Caton et al., Proc. Natl. Acad. Sci. 87(16):6450-54, 1990). Winter and co-workers used a plasmid vector to clone immunoglobulin domains by the polymerase chain reaction for expression in bacteria (Orlandi et al., Proc. Natl. Acad. Sci. 86(10):3833-37, 1989).

Newly developed *E. coli* antibody cloning systems are very useful for the identification of genes encoding desired binding specificities. However, antibodies produced in *E*. *coli* are not generally useful for therapeutic applications. Typically, only the antibody antigen binding fragments, Fab or Fv, can be produced as secreted products in bacteria. In the rare instance when a whole chain tetrameric IgG has been produced in *E. coli,* the C_{H2} domains are not glycosylated. Nonglycosylated antibodies lack the cytolytic activities antibody-directed cellular cytotoxicity (ADCC) and complement activation that make passive immunotherapy so powerful. Mammalian expression systems produce glycosolated antibody and thus circumvent this limitation of the bacterial system. However, recent modifications in the CBER division of the FDA's "Points to Consider" clearly signal their concerns about viral loads associated with monoclonal antibodies produced in mammalian cells. Moreover, it is expected that any engineered antibody produced in a mammalian expression system will be quite expensive ($1500-$5000 per dose). Alternative expression systems that circumvent the difficulties encountered with current mammalian and bacterial systems are therefore highly desirable.

The baculovirus expression system is an attractive alternative to antibody production in *E*. *coli* and mammalian cells. The expression of recombinant proteins using the baculovirus system has been demonstrated in the past several years and has emerged as an excellent choice for high yield production (1-100 mg/L) of biologically active proteins in eukaryotic cells. The baculovirus/insect cell system also circumvents the solubility problems often encountered when recombinant proteins are overexpressed in prokaryotes. In addition, insect cells contain the eukaryotic post-translational modification machinery responsible for correct folding, disulfide formation, glycosylation, β-hydroxylation, fatty acid acylation, prenylation, phosphorylation and amidation not present in prokaryotes. The production of a functional, glycosylated monoclonal antibody recognizing human colorectal carcinoma cells from a baculovirus expression system has been recently demonstrated (Nesbit, J. Immunol. Methods 151:201-208, 1992). Additionally, expression of recombinant IgA has also been demonstrated in baculovirus cells, and this IgA was correctly assembled into heavy chain/light chain heterodimers, N-glycosylated, and secreted (Carayannopoulos et al., Proc. Natl. Acad. Sci. 91:8348-52, 1994, PCT Publication No. WO 98/30577, U.S. Patent No. 6,063,905). Caspar et al. (1997) Blood, 90(9), 3699-3706 mentions the production of idiotype protein in a baculovirus/insect cell system both as a human IgGκ and a single-chain variable fragment (scFv) GM-CSF fusion protein.

### SUMMARY OF THE INVENTION

The present invention provides the use of a first chimeric protein and a second chimeric protein for the manufacture of a composition for therapy of a B cell lymphoma in a human patient,
wherein said first chimeric protein comprises at least a portion of a V_{H} region and at least a portion of an IgM immunoglobulin constant region; and
wherein said second chimeric protein comprises at least a portion of a V_{L} region and at least a portion of a second immunoglobulin constant region;
wherein said manufacture comprises producing said chimeric proteins by a method comprising:
(a) isolating a gene encoding said portion of said V_{H} region from B cells associated with said patient's B cell lymphoma;
(b) inserting said gene encoding said portion of said V_{H} region and a gene encoding said portion of said IgM immunoglobulin constant region into an expression vector to allow the expression of said first chimeric protein;
(c) isolating a gene encoding said portion of said V_{L} region from B cells associated with said patient's B cell lymphoma;
(d) inserting said gene encoding said portion of said V_{L} region and a gene encoding said portion of said second immunoglobulin constant region into an expression vector to allow the expression of said second chimeric protein; and
(e) producing said chimeric proteins by introducing expression vector into an insect cell line;
wherein the composition further comprises a carrier protein conjugated to said chimeric protein.

in said second chimeric protein The immunoglobulin constant region a κ or λ light chain or portion thereof. Examples of second chimeric proteins include V_{L}-κ, and V_{L}-λ.

The present disclosure also provides a method for producing chimeric proteins using recombinant DNA technology and an expression system. This method includes the following steps: (a) isolating a gene encoding the lymphoma V_{H} region of an immunoglobulin chain from B cells of a patient having a B cell lymphoma, (b) inserting the isolated gene encoding the V_{H} region of an immunoglobulin chain and the gene encoding an immunoglobulin IgM constant region into an expression vector to allow the expression of a chimeric protein, (c) producing the chimeric protein by introducing the expression vector into insect cell lines and allowing its expression, and (d) isolating the chimeric protein. The method for producing chimeric proteins further includes a step of inserting a gene encoding V_{L} region of an immunoglobulin chain and a gene encoding a second immunoglobulin constant region into the expression vector to allow the expression of the second chimeric protein.

In preferred embodiments, either or both of the chimeric proteins comprise at least a portion of a V_{H} and/or V_{L} region of an immunoglobulin chain, plus a linker region, and at least a portion of an immunoglobulin constant region.

In one of the embodiments of the invention, the expression vector used to express the chimeric proteins is a baculovirus vector. The vector preferably contains two expression cassettes each having a promoter, a secretory signal sequence and a chimeric protein. One expression cassette contains the baculovirus AcNPV p10 promotor linked to the honey bee melittin secretory signal sequence. The other expression cassette has the polyhedrin promotor linked to a human placental alkaline phosphatase secretory signal sequence. In addition to the listed promoters and signal sequences, other promoters and signal sequences known to those skilled in the art could be used. In some preferred embodiments, the signal sequences are endogenous signal sequences associated with the V_{H} and V_{L} genes isolated from patients, or other signal sequences involved in antibody production. The genes encoding the V_{H} or V_{L} portions of the immunoglobulin chains, and the genes encoding immunoglobulin constant region are inserted, separately and/or together, into the above expression cassette of the baculovirus vector allowing expression of one or two chimeric proteins. In a preferred embodiment, the constant region of the immunoglobulin heavy chain is controlled by the polyhedrin promotor.

Chimeric proteins produced are purified using affinity columns with anti-immunoglobulin antibodies or Ig-binding proteins, such as protein A for the constant region of an immunoglobulin heavy chain, and protein L for kappa light chains, and/or any other proteins that bind to an immunoglobulin binding domain.

The present invention also contemplates covalently coupling the chimeric proteins to a carrier protein such as keyhole limpet hemocyanin (KLH). The composition may also be administered into a patient together with a cytokine such as granulocyte-macrophage-CSF (GM-CSF), or a chemokine such as a monocyte chemotactic protein 3 (MCP 3). Because the present composition containing chimeric protein(s) is specifically related to a particular immunoglobulin from B cells of a patient having B cell lymphoma, administration of this composition induces an immune response against the disease specific idiotype in which particular V_{H} or V_{L} segments are involved. Thus, the administration of the composition alters a B cell lymphoma in a patient. The administration routes for the composition include but are not limited to oral delivery, inhalation delivery, injection delivery, transdermal delivery, and the like.

### BRIEF DESCRIPTION OF THE DRAWINGS

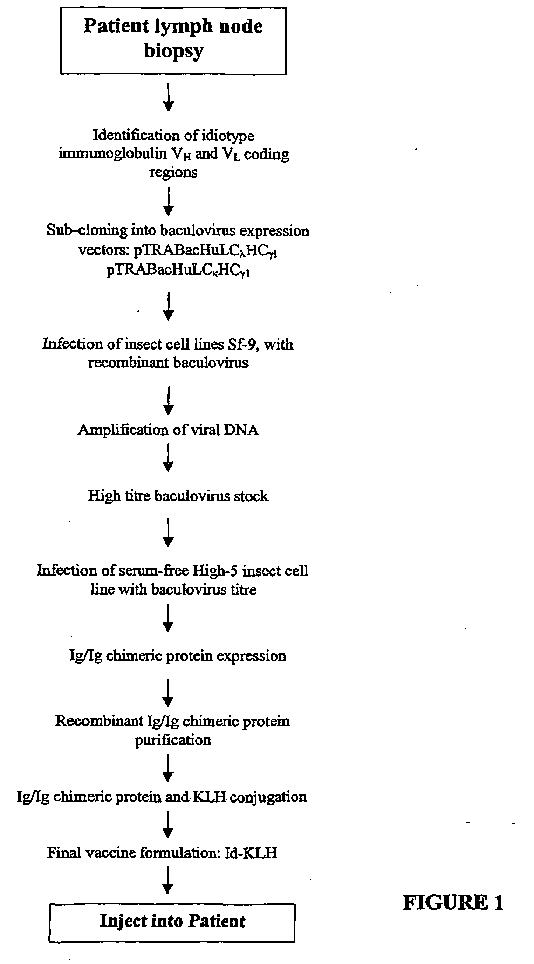

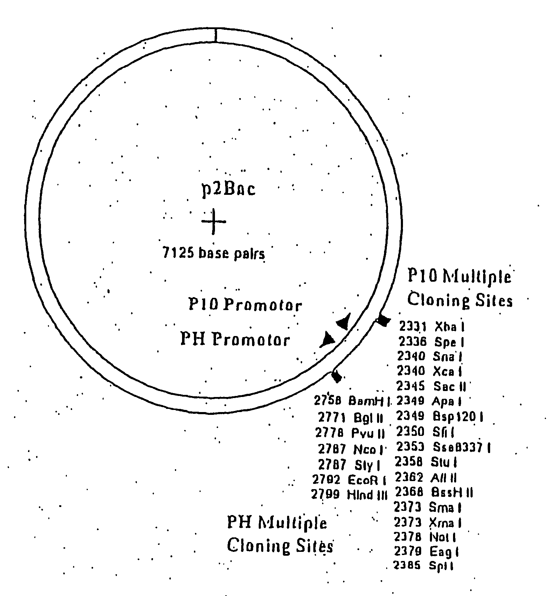

Figure 1: A general scheme for producing a composition comprising chimeric proteins for V_{H} or V_{L} regions of a particular immunoglobulin from B cells from a patient having B cell mediated pathology.
Figure 2: Plasmid map of a baculovirus expression vector p2Bac with multiple cloning sites.

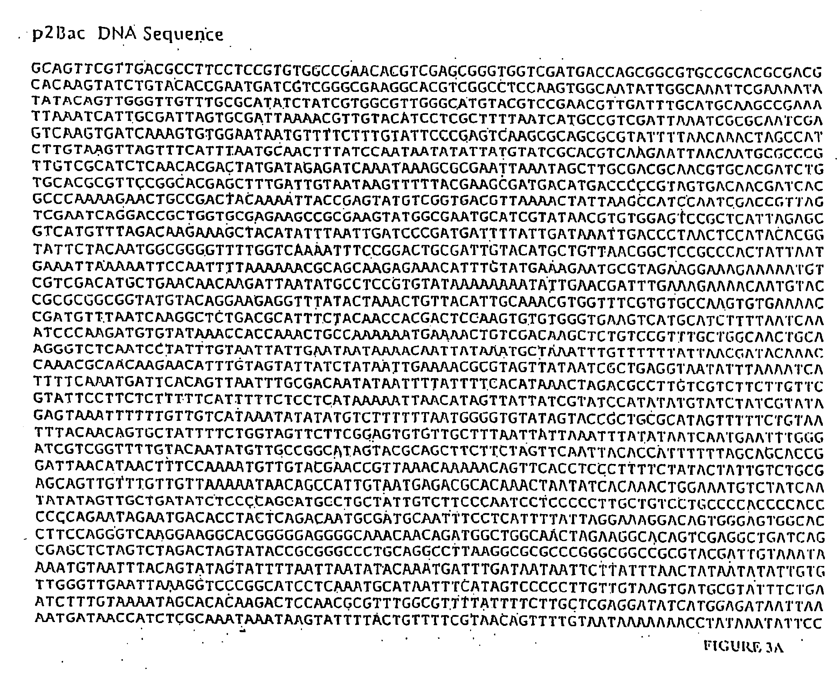

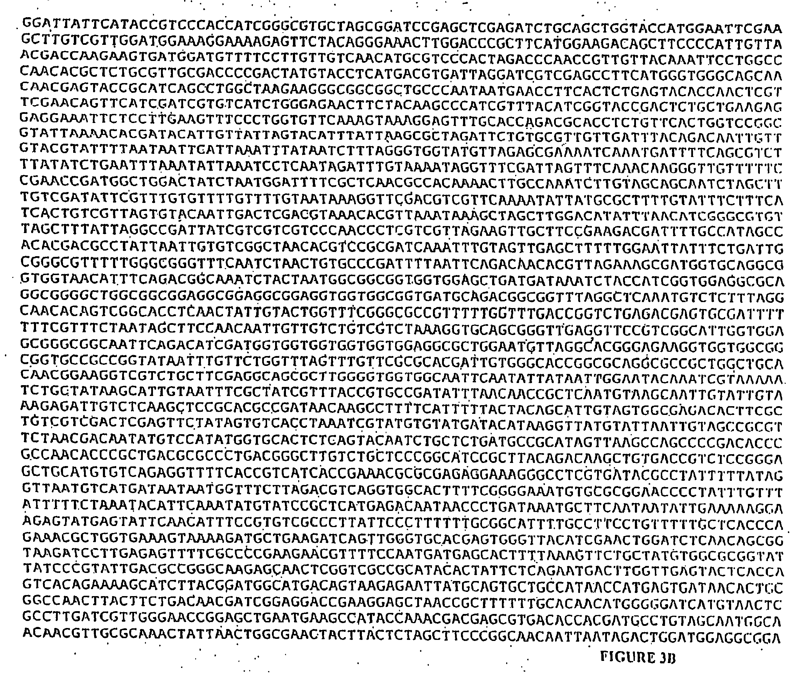

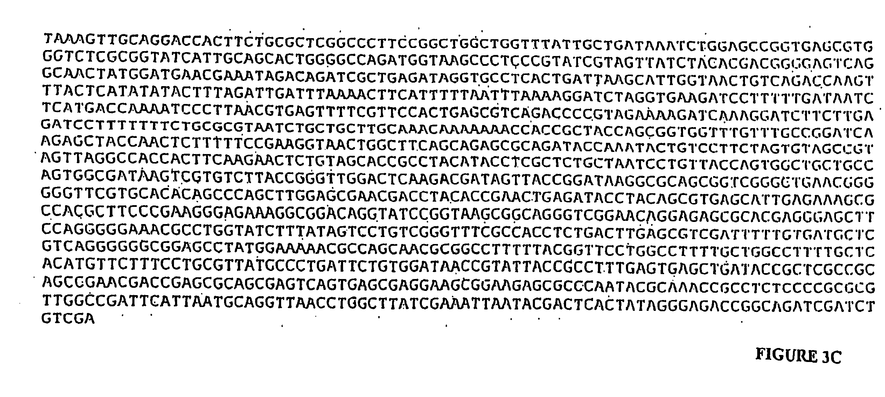

Figure 3: DNA sequence of baculovirus expression vector p2Bac (SEQ ID NO:5). The sequence is depicted from 5' to 3'. The p2Bac vector contains the AcNPV polyhedrin gene promoter (nucleotides 1 to 120 of the GenBank accession number X06637 (SEQ ID NO:92)) and the AcMNPV p10 promoter (nucleotides 8 to 237 of GenBank accession number A28889 (SEQ ID NO:93)).

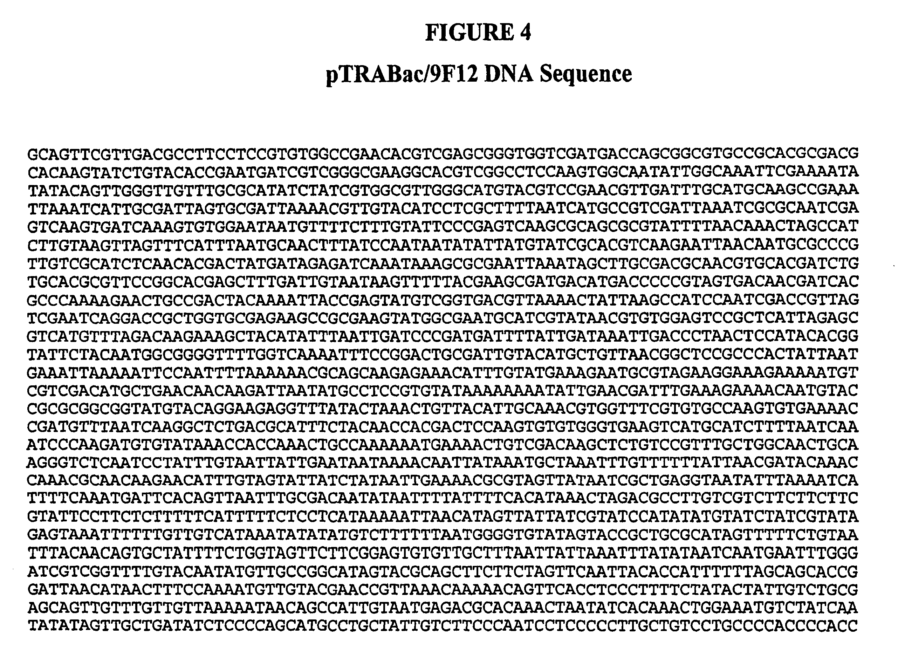

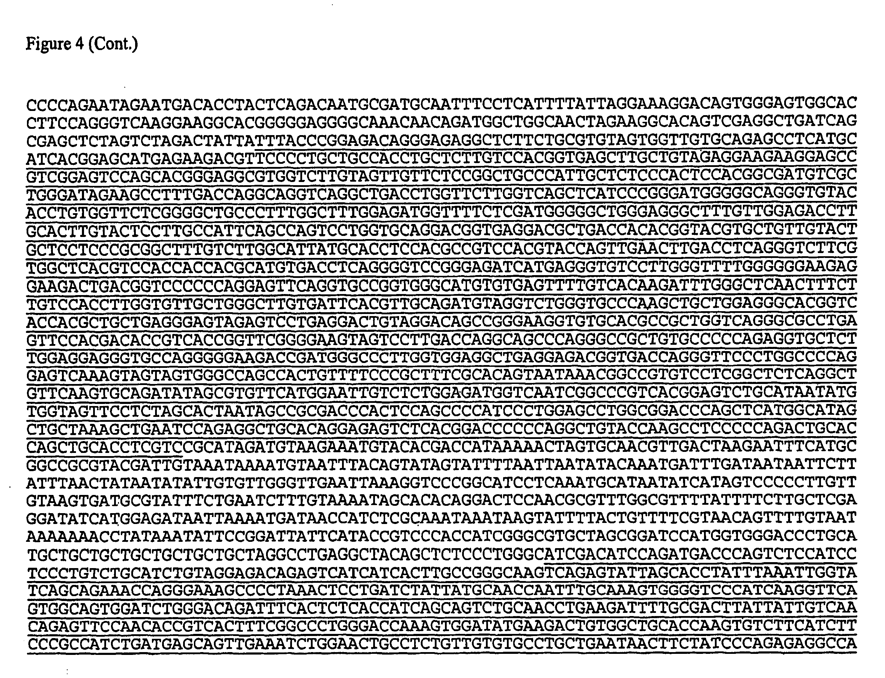

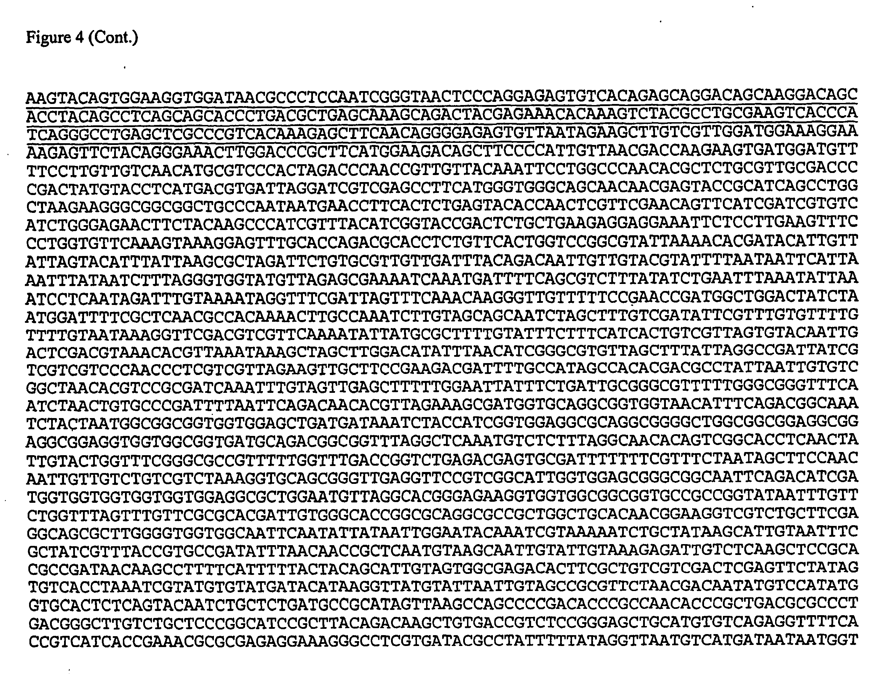

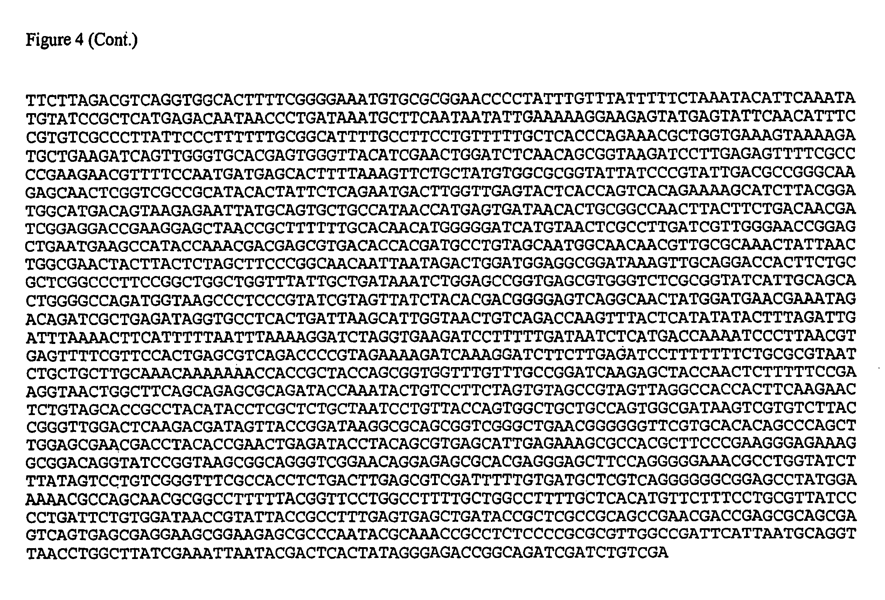

Figure 4: DNA sequence of the plasmid pTRABac/9F12. This plasmid contains the genes for the heavy and light (κ) chains expressed by the stable human cell-line 9F12. This cell line produces a human IgG1/κ antibody specific for tetanus toxoid (SEQ ID NO:89). The underlined regions represent sequences encoding mature 9F12 IgG₁ (TTTACCC....) and kappa (ATCGACA...) chains, respectively. The sequence is depicted from 5' to 3'.
Figure 5a: Plasmid map of recombinant baculovirus expression vector pTRABacHuLC_{κ}HC_{γ1} with IgG_{γ1} constant regions.
Figure 5b: Plasmid map ofrecombinant baculovirus expression vector pTRABacHuLC_{λ}HC_{γ1} with IgG_{γ1} constant regions.
Figure 6A: DNA sequence of pTRABacHuLC_{κ}HC_{γ1} (SEQ ID NO:6). The sequence is depicted from 5' to 3'.
Figure 6B: DNA sequence of pTRABacHuLC_{λ}HC_{γ1} (SEQ ID NO:7). The sequence is depicted from 5' to 3'.
Figure 6C: DNA sequence of pTRABacHuLC_{κ}HC_{γ1} following modification utilizing the kappa stuff primers (SEQ ID NO:90). The sequence is depicted from 5' to 3'.
Figure 6D: DNA sequence of pTRABacHuLC_{λ}HC_{γ1} following modification utilizing the lambda stuff primers (SEQ ID NO:91). The sequence is depicted from 5' to 3'.

### DETAILED DESCRIPTION OF THE PREFERRED EMBODIMENTS

The possibility of evoking an immune response that would recognize and eliminate neoplastic cells while sparing normal tissue represents an exciting approach to the treatment of cancer. Inducing such an immune response is assisted by identifying a unique tumor antigen. B-cell malignancies express a unique antigen, the immunoglobulin idiotype (Id), on their surface. This antigen contains protein sequences from both the variable immunoglobulin heavy and light regions (V_{H} and V_{L}). Each B-cell harbors a unique genetic sequence used in the production of the immunoglobulin idiotype. Consequently, as most B cell malignancies arise from the clonal expansion of a single B cell, all cells comprising a B-cell malignancy expresses a unique Id protein. Hence, idiotypic protein should serve as an ideal target for immune-based therapy of any B cell malignancy, such as lymphoma or leukemia.

### Passive Immunotherapy

Early immunotherapy strategies focused on the use of monoclonal antibodies against tumor-specific idiotype (anti-Id MoAb). This approach resulted in tumor regression and long-lasting remissions in several patients with non-Hodgkin's lymphoma. However many patients experienced eventual relapse (Miller et al., N. Engl. J. Med. 306(9):517-22, 1982; Maloney et al., Blood 80(6):1502-10, 1992; Brown et al., Blood 73(3):651-61, 1989; Brown et al., Semin. Oncol. 16(3):199-210, 1989; Meeker et al., Blood 65(6):1349-63,1985.

One difficulty that arose in the studies described above was that cells of a malignant B or T cell lymphoma could alter their expression of their idiotypic immunoglobulins or T cell receptors. Two examples of this were described in some of the articles listed above (Cleary *et al.,* 1986, and Meeker *et al.,* 1985). It was also shown that T cell leukemia cells could escape anti-idiotypic antibodies by reducing their expression of surface T cell receptor (Maecker et al., J Immunol. 141:2994-3002, 1985), and this was confirmed for B cell leukemias in an animal model (Stevenson et al., J.Immunol. 130(2):970-3,1983). Other studies demonstrated that that there is idiotypic variation even within a given human B cell lymphoma (Berinstein et al., J.Immunol. 144(2):752-8, 1990; Levy S, et al., J. Exp. Med. 168(2):475-89,1988;). Such mutations appeared responsible for the decreased effectiveness of the anti-Id MoAb over time (Berinstein *et al., supra*; Tao et al., Nature 362(6422):755-8,1993; Chen et al., J.Immunol. 153(10):4775-87, 1994).

One way to avoid this problem is via the generation and use of a polyclonal antisera against the idiotypic protein. Caspar and co-workers studied the potential of a polyclonal antibody-based therapy in a mouse model system (Caspar et al., Blood 90:3699-706, 1997). These authors vaccinated a mouse using idiotypic proteins from a non-Hodgkin's lymphoma patient who had relapsed following successful monoclonal antibody therapy. The resultant polyclonal antibodies recognized idiotypic proteins from both the original tumor and all variants. Therefore, generation of polyclonal antibody response specific to the idiotype of a B cell lymphoma or leukemia would represent an improvement over monoclonal antibody therapy. Producing sufficient quantities of protein to for a vaccination to produce polyclonal antibodies is a significant burden of this approach.

### Active Immunotherapy

Active immunotherapy may avoid the phenomenon of mutational escape seen with passive immune strategies. Such therapy has the potential to generate a broader immune response and thereby recognize the heterogeneous tumor cell population that can arise over time. The difficulty with active immunotherapy lies in convincing the patient's immune system to react against a perceived "self antigen" expressed by the tumor. As with idiotypic protein, many of the antigens expressed by tumors are weak immunogens.

In the instant invention, the unique specificity of the immune system has been adapted to treat B cell lymphomas. In the instant invention, the DNA sequence encoding the variable region of the idiotypic immunoglobulins was cloned using primers derived from the 5' end of each unique subfamily of light and heavy immunoglobulin chains together with a constant region primer. Typically, this process uses one of several suitable cloning techniques such as PCR. These constant region primers, in combination with one for the V_{H} region and one for the V_{L} region, may be used to clone the variable regions as a first step in producing a chimeric protein comprising a variable region and a constant region. Alternatively, techniques such as 5' RACE may be used. In the case of one patient described *infra,* 5' RACE was used to clone the variable regions of the heavy and light immunoglobulin chains in order to produce a chimeric protein. Examples of chimeric proteins include: V_{L} / C_{κ}, V_{L} / C_{λ}, V_{H} / C_{κ}, and V_{H} / C_{λ}. These chimeric proteins are produced in insect cells using a baculovirus vector. The chimeric protein thus comprises a portion of a variable region from an immunoglobulin molecule from a patient and also comprises a portion of a constant region from a source other than the patient. These chimeric proteins are predicted to be more efficiently produced than using existing systems for producing idiotypic proteins and will be excellent immunogens for use in vaccination protocols.

The present invention fills the great demand for an effective treatment for B cell lymphomas. The invention takes advantage of the unique cell surface antigens present on the surface of B cells involved in B cell lymphomas, and are prepared in a patient-specific manner. Such vaccines provide exquisite selectivity by being tailored to the markers unique to the pathogenic B cells found in a given patient.

The baculovirus/insect cell expression system has proven effective for the efficient production of functional antibodies for immunotherapy from any given patient. This baculovirus expression vector described below in the Experimental section was designed such that only two custom gene-specific primers were needed to amplify any pair of antibody variable regions for easy subcloning and expression as human kappa light chain and IgG_{γ1} heavy chain. The incorporation of heterologous secretary signal sequences, which directed the heavy and light chains to the secretary pathway, were incorporated for the expression of large amounts of active immunoglobulin from insect cells. This vector should be useful for the expression of any kappa light chain variable region (V_{L}) in frame with human kappa constant region and secreted via the human placental alkaline phosphatase secretary signal sequence; and any heavy chain variable region (V_{H}) in frame with the human IgG_{γ1} constant domain led by the honey bee melittin secretary signal sequence. In other systems, the lambda light chain constant region replaces the kappa constant region. The chimeric protein is then expressed with the V_{L} region in frame with human lambda constant region and secreted via the human placental alkaline phosphatase secretary signal sequence, along with any heavy chain variable region (V_{H}) in frame with the human IgG_{γ1} constant domain led by the honey bee melittin secretary signal sequence. Any monoclonal antibody, mouse or human, either from a monoclonal cell line or identified by phage display cloning, could be easily expressed as whole human IgG_{γ1}/κ or IgG_{γ1}/λ in this vector after two simple subcloning steps. Additionally, different immunoglobulin types, including IgM heavy chains, or segments thereof, could be used in place of IgG_{γ1}. Furthermore, besides those signal sequences described *supra,* the instant invention may use other secretory signal sequences such as the endogenous secretory sequences associated with the immunoglobulin genes derived from a given patient. Additionally, one of skill in the art would be able to select several different primers that could be used equivalently in this system to produce equivalent results to amplify any pair of antibody variable regions for easy subcloning.

In some instances, utilization of the baculovirus system for the expression of biologically active proteins has been hampered by the inability to efficiently solubilize recombinant proteins without excessive proteolytic degradation. In order to circumvent solubility and proteolysis problems encountered with the expression of recombinant proteins in insect cells, baculovirus transfer vectors were developed for the efficient secretion of biologically active proteins. These vectors that facilitate the secretion of recombinant proteins from host insect cells are constructed by inserting functional secretory leader sequences downstream of the polyhedrin promoter. In-frame insertion of cDNA sequences resulted in the synthesis of proteins containing a heterologous signal sequence which directed the recombinant protein to the secretory pathway. Human and insect leader sequences were both tested to maximize secretion of heterologous proteins from insect cells. The human placental alkaline phosphatase signal sequence (SEQ ID NO:1: MLGPCMLLLLLLLGLRLQLSLG; DNA sequence is SEQ ID NO:2: ATG GTG GGA CCC TGC ATG CTG CTG CTG CTG CTG CTG CTA GGC CTG AGG CTA CAG CTC TCC CTG GGC) and the honeybee melittin signal sequence (SEQ ID NO:3: MKFLVNVALVFMVVYISYIYA; DNA sequence is SEQ ID NO:4: ATG AAA TTC TTA GTC AAC GTT GCA CTA GTT TTT ATG GTC GTG TAC ATT TCT TAC ATC TAT GCG) have both proved useful for the secretion of numerous bacterial and human proteins (Mroczkowski et al., J Biol. Chem. 269:13522-28, 1994 and Tessier et al., Gene 98:177-83,1991).

To tailor the present invention to a particular patient first requires identification and isolation of the genes encoding the unique antigens, and then the means of producing those antigens. This may be accomplished in a number of different ways available to one of skill in the art. For example, a recently developed method that is adapted to the needs of the instant invention uses a novel baculovirus/insect cell expression system and was recently developed for the efficient production of functional antibodies for immunotherapy (see U.S. Provisional Application Serial No. 60/244,722, entitled "Expression Vectors for Production of Recombinant Immunoglobulin").

Expression of recombinant proteins using the baculovirus system allows the production of large quantities of biologically active proteins without many of the drawbacks associated with proteins made in bacteria, and also avoids the complications of using mammalian cells. For example the immunoglobulin genes from the stable human cell-line 9F12 (ATCC#HB8177), which produces a human IgG1/κ antibody specific for tetanus toxoid, were cloned into a baculovirus dual promoter expression transfer vector. Intact IgG1/κ immunoglobulin was produced in insect cells that behaved similarly to the mammalian antibody in SDS-PAGE analysis and Western blots. The antibody produced by insect cells was glycosylated. The binding affinities of purified Mab9F12 and purified baculovirus expressed antibody were determined to be identical and production levels were determined to be approximately 5-10 µg/ml.

Soluble human immunoglobulin fragments containing specific epitopes of the particular variable regions can be produced in insect host cells via genetic engineering. These soluble recombinant immunoglobulin proteins containing patient-derived particular V_{H} and/or V_{L} regions can be used as a therapeutic composition. When administered into the patient, it would specifically induce, in vivo, a cell mediated immune response for altering the B cell mediated pathology.

This technology has also been applied towards the rapid identification and cloning of patient-specific V_{α} and Vβ genes expressed by a T cell lymphoma, then expressing these as recombinant κ/V_{α} or IgGγ₁/V_{β} molecules in insect cells (see U.S. Provisional Application Serial No. 60/266,133 entitled "Method and Composition for Altering a T Cell Mediated Pathology"). Molecules produced by this method were formulated and used to induce anti-idiotypic cell-mediated immunity against lymphomas in a patient-specific fashion.

The term "altering" or "alters" refers to the ability of a compound or composition to modulate a B cell mediated pathology. A compound which alters a B cell pathology may do so by a number of potential mechanisms, including raising antibodies directed at the compound which in turn destroys cells of the B cell pathology, inducing apoptosis in the B cells involved in the pathology, inhibiting further growth and division of cells of the B cell pathology, inducing cell-mediated immunity directed at the cells of the B cell pathology, or otherwise inhibiting the activity of the pathological B cells. The exact mechanism that causes the alteration need not be determined, but only that an alteration in the B cell mediated pathology occurs by some mechanism as a consequence of adding the inventive molecules or compositions.

The term "B cell mediated pathology" or "B cell pathology" refers to those diseases and conditions that arise from inappropriate replication or activity of B cells. In the invention, the B cell mediated pathology is a B cell lymphoma that results from inappropriate replication of B cells. B cell lymphomas are difficult to treat effectively with the currently available medical methods. Other types of B cell pathologies which involve inappropriate replication ofB cells include chronic and acute B cell leukemias, multiple myelomas, and some non-Hodgkin's lymphomas.

The term "B cell" refers to a cell of the immune system of an organism which is involved in the humoral immunity in normal functioning of a organism (i.e., one that is not experiencing a B cell mediated pathology). B cells are white blood cells that develop from bone marrow and produce antibodies; they are also known as B lymphocytes. In general, B cells are cells involved in antibody production in an organism.

The term "pathology" refers to a state in an organism (*e.g.*, a human) which is recognized as abnormal by members of the medical community. The lymphoma to be treated in the present invention is characterized by an abnormality in the function of B cells.

The term "patient" refers to an organism in need of treatment for a pathology, or more specifically, a B cell pathology. The term refers to a living subject who has presented at a clinical setting with a particular symptom or symptoms suggesting the need for treatment with a therapeutic agent. The treatment may either be generally accepted in the medical community or it may be experimental.

A patient's diagnosis can alter during the course of disease progression, either spontaneously or during the course of a therapeutic regimen or treatment.

The term "chimeric protein" refers to a protein which comprises a single polypeptide chain comprising segments derived from at least two different proteins. The segments of the chimeric protein must be derived from heterologous proteins, that is, all segments of the chimeric polypeptide do not arise from the same protein. The chimeric proteins used in the present invention include proteins containing portions of the V_{H} or V_{L} region of an immunoglobulin chain, but do not comprise the entire C region of those chains as found in the B cell clone from which the V_{H} or V_{L} regions is derived. Furthermore, the V_{H} or V_{L} region may not include the entire variable region, but does include enough to generate an immune response. Chimeric proteins used in the present invention may also include proteins in which a segment of the naturally occurring protein has been replaced with an equivalent naturally or non-naturally occurring segment. This includes immunoglobulin constant regions in which a segment of the protein has been replaced with a linker, segment or domain that is partially or entirely manmade. In all cases, however, the gene for the chimeric protein of the instant invention will not be the same as the gene for the immunoglobulins which occur naturally in the patient The gene for the chimeric protein will be distinguishable from naturally occurring protein for one of the following reasons: (1) it will not be the full length immunoglobulin gene or cDNA from the patient or (2) it will be a different subtype than isolated from the patient.

The terms "protein," "polypeptide," and "peptide" are used herein interchangeably.

The term "naturally" or "native" refers to a protein as it is isolated from nature. Thus, a naturally occurring protein may refer to a protein as it is found in nature which is encoded by a gene that has not been modified by the use of recombinant techniques. A native protein may refer to a protein as it may be found or synthesized in nature. These terms may also apply to proteins which are produced by biological system such as the bacculovirus virus system of the present invention or by the culture of cells derived from patients. A native protein may alternately refer to an isolated protein which has not been denatured. The term "native" may also refer to the manner in which polypeptide or protein is folded, either alone or in combination with other polypeptides, so that it resembles similar proteins found in nature, or how it is modified after translation ("post-translational modifications") so that it resembles similar proteins found in nature. A naturally-occurring protein may be found only in pathological B cells from a single patient, nevertheless, this may be considered a naturally-occurring protein.

The term "segment" or "portion" is used to indicate a polypeptide derived from the amino acid sequence of the proteins used for the chimeric proteins having a length less than the full-length polypeptide from which it has been derived. It is understood that such segments may retain one or more characterizing portions of the native polypeptide. Examples of such retained characteristics include: binding with an antibody specific for the native polypeptide, or an epitope thereof.

The terms "V_{H}" and "V_{L}" refer to the variable regions of the polypeptide chains of immunoglobulin molecules, or nucleic acids encoding such polypeptide chains. One skilled in the art realizes the meaning of these terms. The exact sequence of a variable region cannot be predicted and must be determined by isolating the sequence in question. The V_{H} and V_{L} regions isolated from particular patients are used in the instant invention. The exact sequence of a kappa (κ) or lambda (λ) light chain is determined by clonal rearrangements of the V regions, J regions and Constant region of the light chain locus. (The kappa and lambda loci are separate and distinct.) The exact sequence of a heavy chain is determined by clonal rearrangements of the V regions, D regions, J regions and Constant region of the heavy chain locus. Additional sequence variation in the variable region arises from imprecision during the recombination process and also is generated by somatic mutations subsequent to the end of the recombination process. The terms "V_{H}" and "V_{L}" also refer to portions or segments of the V_{H} and V_{L} regions. A segment of the V_{H} and V_{L} region may also include all or substantially all of the V region. The term "substantially all" refers to approximately 90% of the entire variable region, or approximately 80% of the entire variable region. The portion of the V_{H} and V_{L} region present must be sufficient to allow the chimeric molecule to operate in the present invention. The terms "V_{H}" and "V_{L}" also refer to functional derivatives of such polypeptide regions as described *infra*.

The term "immunoglobulin constant region" refers to all or part of that portion of immunoglobulin molecules which are not encoded by the variable regions of immunoglobulins. The term "immunoglobulin constant region" may also refer to the DNA sequence encoding the immunoglobulin constant region. The immunoglobulin constant region includes the segments C_{L}, C_{H1}, C_{H2}, C_{H3}, and the Hinge region. Immunoglobulin types include IgG_{γ1}, IgG_{γ2}, IgG_{γ3}, IgG_{γ4}, IgA₁, IgA₂, IgM, IgD, IgE heavy chains, and κ or λ light chains or segments thereof.

The term "immunoglobulin fold" or "immunoglobulin domain" refers to a structural element of the immunoglobulin super family. The immunoglobulin domain is a conserved, repeating structural domain of approximately 110 amino acids each.

Immunoglobulin domains are found in many protein molecules, including antibodies, the T cell antigen receptor, cytokine receptors (*e.g.*, the platelet-derived growth factor receptor with 5 Ig domains), cell adhesion molecules (*e.g.*, ICAM-1/CD54), and many others. Two immunoglobulin domains are found in each TCR; one in the variable region and one in the constant region. Two immunoglobulin domains are found in antibody light chains and four are found in IgG heavy chains. The present invention contemplates the replacement of one or two domains of the constant region with domains from a different molecule, such as an immunoglobulin molecule, to produce a modified (chimeric) constant region which may have different properties such as binding to other molecules.

The terms "IgG₁, IgG₂, IgG₃, IgG₄, IgA, IgA₁, IgA₂, IgM, IgD, IgE" refer to classes and subclasses of human immunoglobulins. The terms may refer to either the DNA sequences or the amino acid sequences of the proteins. The class and subclass of an immunoglobulin molecule is determined by its heavy chain. IgG and IgD are different classes of immunoglobulins; IgG₁ and IgG₂ are different subclasses of immunoglobulin molecules. The term "IgA" may refer to any subclass of IgA molecules, including and IgA₂ molecules. In some embodiments, the immunoglobulin heavy chain used may be a chimeric protein that contains amino acids from a second protein.

The term "IgG_{γ1}" refers to the heavy chain associated with the IgG₁ class of immunoglobulins. IgG₁ represents approximately 66% of human IgG immunoglobulins (Roitt et al., Immunology, Mosby, St. Louis, pg. 4.2, 1993).

The terms "kappa constant region," "lambda constant region," "κ constant regions," and "λ constant region" refer to the constant regions of kappa (κ) and lambda (λ) light chains that remain constant during the development of the immune system. The terms may refer to either the DNA sequences or the amino acid sequences of the proteins. In some embodiments, portions of the immunoglobulin light chain may be comprised in a chimeric protein that contains amino acids from one or more other proteins.

The term "administering" relates to a method of contacting a compound with or into cells or tissues of an organism. The B cell mediated pathology can be prevented or treated when the cells or tissues of the organism exist within the organism or outside of the organism. Cells existing outside the organism can be maintained or grown in cell culture dishes. For cells harbored within the organism, many techniques exist in the art to administer compounds, including (but not limited to) oral, parenteral delivery, dermal application, injection, and aerosol applications.

The term "composition" refers to a mixture that contains the protein of interest. In preferred embodiments, the composition may contain additional components, such as adjuvants, stabilizers, excipients, and the like.

The term "associated with" in reference to the relation of a variable region to a B cell clone refers to the variable region that is found on the immunoglobulins produced by a particular B cell clone.

The term "B cell clone" refers to the clonal descendants of a single B cell. Clonal descendants ofB cells express the same idiotype in the produced antibodies as the parental cell. One skilled in the art realizes that clonal descendants of a B cell may have undergone somatic mutation within the variable region of the immunoglobulin gene but still remain part of the B cell clone.

The term "isolating" refers to removing a naturally occurring nucleic acid sequence from its normal cellular environment. Thus, the sequence may be in a cell-free solution or placed in a different cellular environment. The term does not imply that the sequence is the only nucleotide chain present, but that it is essentially free (about 90 - 95% pure at least) of non-nucleotide material naturally associated with it, and thus is distinguished from isolated chromosomes. Also, by the use of the term "isolating" in reference to nucleic acid is meant that the specific DNA or RNA sequence is increased to a significantly higher fraction (2- to 5-fold) of the total DNA or RNA present in the solution of interest than in the cells from which the sequence was taken. This could be caused by a person by preferential reduction in the amount of other DNA or RNA present, or by a preferential increase in the amount of the specific DNA or RNA sequence, or by a combination of the two. However, it should be noted that enriched does not imply that there are no other DNA or RNA sequences present, just that the relative amount of the sequence of interest has been significantly increased. The term "significant" is used to indicate that the level of increase is useful to the person making such an increase, and generally means an increase relative to other nucleic acids of about at least 2-fold, more preferably at least 5- to 10-fold or even more. The term also does not imply that there is no DNA or RNA from other sources. The DNA from other sources may, for example, comprise DNA from a yeast or bacterial genome, or a cloning vector such as pUC19. This term distinguishes from naturally occurring events, such as viral infection, or tumor-type growths, in which the level of one mRNA may be naturally increased relative to other species of mRNA. That is, the term is meant to cover only those situations in which a person has intervened to elevate the proportion of the desired nucleic acid.

Isolated DNA sequences are relatively more pure than in the natural environment (compared to the natural level this level should be at least 2- to 5-fold greater, e.g., in terms of mg/mL). Individual sequences obtained from PCR may be purified to electrophoretic homogeneity. The DNA molecules obtained from this PCR reaction could be obtained from total DNA or from total RNA. These DNA sequences are not naturally occurring, but rather are preferably obtained via manipulation of a partially purified naturally occurring substance (e.g., messenger RNA (mRNA)). For example, the construction of a cDNA library from mRNA involves the creation of a synthetic substance (cDNA) and pure individual cDNA clones can be isolated from the synthetic library by clonal selection from the cells carrying the cDNA library. The process which includes the construction of a cDNA library from mRNA and isolation of distinct cDNA clones yields an approximately 10⁶-fold purification of the native message. Thus, purification of at least one order of magnitude, preferably two or three orders, and more preferably four or five orders of magnitude is expressly contemplated.

The term "gene encoding" refers to a sequence of nucleic acids which codes for a protein or polypeptide of interest. The nucleic acid sequence may be either a molecule of DNA or RNA. In preferred embodiments, the molecule is a DNA molecule. In other preferred embodiments, the molecule is a RNA molecule. When present as a RNA molecule, it will comprise sequences which direct the ribosomes of the host cell to start translation (*e.g*., a start codon, ATG) and direct the ribosomes to end translation (*e.g.,* a stop codon). Between the start codon and stop codon is an open reading frame (ORF). One skilled in the art is very familiar with the meaning of these terms.

The term "insect cell lines" refers to cell lines derived from insects and susceptible to infection by the bacculovirus. One skilled in the art is familiar with such cell lines and the techniques needed to utilize them. Representative examples of insect cell lines include *Spodoptera frugiperda (sf9)* and *Trichoplusia n*i (Hi-5) cell lines.

The terms "*Trichoplusia ni* (High-5) cells" and "*Spodoptera frugiperda (sf9)* cells" refers to insect cell lines used in combination with baculovirus expression vectors. One skilled in the art is familiar with these cell lines and how to obtain them.

The term "inserting" refers to a manipulation of a DNA sequence via the use of restriction enzymes and ligases whereby the DNA sequence of interest, usually encoding the gene of interest, can be incorporated into another nucleic acid molecule by digesting both molecules with appropriate restriction enzymes in order to create compatible overlaps and then using a ligase to join the molecules together. One skilled in the art is very familiar with such manipulations and examples may be found in Sambrook *et al.* (Sambrook, Fritsch, & Maniatis, "Molecular Cloning: A Laboratory Manual", 2nd ed., Cold Spring Harbor Laboratory, 1989), which is hereby incorporated by reference in its entirety including any drawings, figures and tables.

The term "adjuvant" refers to a substance which is provided with the antigen or immunogen of choice, *e.g.*, the protein or polypeptide to which an immune response is desired, to enhance the immune response when one attempts to raise an immune response in an animal against the antigen or immunogen of choice. One skilled in the art is familiar with appropriate adjuvants to select and use. Adjuvants approved for human use include aluminum salts and MF59 (Singh and O'Hagan, Nature Biotech 17:1075-81, 1999). Other adjuvants are being developed (*Id*.) and may be used in conjunction with the present invention.

The term "keyhole-limpet hemocyanin" or "KLH" refers to a protein which is isolated from keyhole limpets which is commonly used as a carrier protein in the immunization process. One skilled in the art is familiar with the meaning of the term keyhole limpet hemocyanin.

The term "cytokine" refers to a family of growth factors, soluble (glyco)protoins, secreted primarily from leukocytes. Cytokines stimulate both the humoral and cellular immune responses, as well as the activation of phagocytic cells. Cytokines are synthesized, stored and transported by various cell types not only inside of the immune system (lymphokines, interleukins, monokines, tumor necrosis factors, interferons) but also by other cells which are associated with the study of hematology (colony-stimulating factors), oncology (transforming growth factors), and cell biology (peptide growth factors, heat shock and other stress proteins).

Cytokines secreted from lymphocytes are termed lymphokines, while those secreted by monocytes or macrophages are referred to as monokines. Many of the lymphokines are also referred to as interleukins (ILs), since they are not only secreted by leukocytes but they are also able to affect the cellular responses of leukocytes. Specifically, interleukins are growth factors targeted to cells of hematopoietic origin.

The term "growth factor" refers to a protein that binds receptors on the surface of a cell and subsequently activates cellular proliferation and/or differentiation. Many growth factors are quite versatile and can act to stimulate cellular division in a wide variety of cell types, while others are specific to a particular cell-type.

The term "chemokine" refers to a group of small proinflammatory cytokines which function as chemoattractants and activators for leukocytes and represent a superfamily of over 30 chemotactic cytokines. They orchestrate the activation and migration of immune system cells from the blood or bone marrow to the site of infection and damaged tissue. Chemokines also play an essential role in the growth and proliferation of primitive stem cells found in bone marrow which in turn develop into mature immune cells. Chemokines are involved in a wide range of acute and inflammatory diseases and exert their action by binding to receptors of the seven-transmembrane-helix class.

Chemokines frequently range from 8 to 11 kDa in molecular weights, are active over a concentration range of 1 to 100 ng/ml, and are produced by a wide variety of cell types. The production of chemokines typically is induced by exogenous irritants and endogenous mediators such as IL-1, TNF-alpha, and PDGF. The chemokines bind to specific cell surface receptors and can be considered second-order cytokines that appear to be less pleiotropic than first-order proinflammatory cytokines because they are not potent inducers of other cytokines and exhibit more specialized functions in inflammation and repair.

The term "granulocyte-macrophage colony-stimulating factor " or "GM-CSF" refers to a small (less than 20 kDa) secreted protein. It binds to specific cell surface receptors and functions as species-specific stimulator of bone marrow cells. It stimulates the growth and differentiation of several hematopoietic cell lineages including dendritic cells, granulocytes, macrophages, eosinophils, and erythrocytes. In particular, this cytokine also plays a role in shaping cellular immunity by augmenting T-cell proliferation (Santoli et al., J.Immunol. 141(2):519-26, 1988), increasing expression of adhesion molecules on granulocytes and monocytes (Young et al., J.Immunol. 145(2):607-15, 1990; Grabstein et al., Science 232(4749):506-08, 1986), and by augmenting antigen presentation (Morrissey et al., J.Immunol 139(4):1113-9, 1986; Heufler et al., J. Exp. Med. 167(2):700-05, 1988; Smith et al., J.Immunol. 144(5):1777-82, 1990).

The term "monocyte chemotactic protein-3" or "MCP-3" refers a chemokine primarily produced by monocytes. MCP-3 has a wide spectrum of chemotactic activity and attracts monocytes, dendritic cells, lymphocytes, natural killer cells, eosinophils, basophils, and neutrophils. The cDNA was cloned in 1993 by Minty et al., Eur Cytokine Netw 4(2):99-110, 1993, and Opdenakker et al., Biochem Biophys Res Commun., 191(2):535-42, 1993. Its properties have been recently reviewed by Proost et al., J Leukoc Biol. 59(1):67-74, 1996.

The term "expression vector" refers to a recombinant DNA construct which is designed to express a selected gene of interest, usually a protein, when properly inserted into the expression vector. One skilled in the art understands the term. Expression vectors commonly include a promotor at the 5' end of the site where the gene of interest is inserted and a terminator region at 3' end of the site. Frequently the gene of interest is inserted into the appropriate site by means of selected restriction enzyme cleavage sites. The term "expression vector" also refers to a DNA construct such as described above into which the gene of interest encoding the product of interest has already been inserted.

The term "baculovirus expression vector" refers to a DNA construct which is designed to express a selected gene when used in the baculovirus system. Any of the potential baculoviruses or expression vectors designed to function in the baculovirus system may be used in the instant invention. In a similar fashion, the term "expression vector" is a genus which encompasses the particular embodiment of baculovirus expression vectors, but "expression vectors" may function in cells and cell lines aside from, or in addition to, insect cell lines.

The term "allow the expression of" refers to placing an expression vector into an environment in which the gene of interest will be expressed. This commonly means inserting the expression vector into an appropriate cell type where the promotor and other regions necessary for gene expression will be recognized by the host cell's components and will cause the expression of the gene of interest. The expression normally consists of two steps: transcription and translation. Expression can also be conducted in vitro using components derived from cells. One skilled in the art is familiar with these techniques, and such techniques are set forth in Sambrook *et al.* (Sambrook, Fritsch, & Maniatis, "Molecular Cloning: A Laboratory Manual", 2nd ed., Cold Spring Harbor Laboratory, 1989). In the preferred embodiment, the expressed product is a protein or polypeptide.

The term "secretory signal sequence" refers to a peptide sequence. When this sequence is translated in frame as a peptide attached to the amino-terminal end of a polypeptide of choice, the secretory signal sequence will cause the secretion of the polypeptide of choice by interacting with the machinery of the host cell. As part of the secretory process, this secretory signal sequence will be cleaved off, leaving only the polypeptide of interest after it has been exported. In preferred embodiments, the honey bee melittin secretory signal sequence is employed. In other preferred embodiments, the human placental alkaline phosphatase secretory signal sequence is employed. The present invention is not limited by these secretory signal sequences and others well known to those skilled in the art may be substituted in place of, and in addition to, these. The term "secretory signal sequence" also refers to a nucleic acid sequence encoding the secretory peptide.

The term "ELISA" refers to "Enzyme-Linked ImmunoSorbent Assay" in which the presence or concentration of a protein is determined by its binding to the plastic well of an ELISA plate followed by its subsequent detection by antibodies specific for the protein to be quantified or detected.

The term "promoter controls" refers to an arrangement of DNA in an expression vector in which a promoter is placed 5' to a gene of interest and causes the transcription of the DNA sequence into an mRNA molecule. This mRNA molecule is then translated by the host cell's machinery. One skilled in the art is very familiar with the meaning of this term.

The terms "protein A," "protein G," and "protein L" refer to specific bacterial proteins which are capable of specifically binding immunoglobulin molecules without interacting with an antigen binding site. Protein A is a polypeptide isolated from *Staphylococcus aureus* that binds the Fc region of immunoglobulin molecules. Protein G is a bacterial cell wall protein with affinity for immunoglobulin G (IgG), which has been isolated from a human group G streptococcal strain (G148). Protein L is an immunoglobulin light chain-binding protein expressed by some strains of the anaerobic bacterial species *Peptostreptococcus magnus.*

The term "B cell lymphoma" refers to a cancer that arises in cells of the lymphatic system from B cells. B cells are white blood cells that develop from bone marrow and produce antibodies. They are also known as B lymphocytes.

The term "refractory low grade B cell lymphoma" refers to a low grade B cell lymphoma that has not responded to treatment. The term "low grade B cell lymphoma" refers to a lymphoma that tends to grow and spread slowly, including follicular small cleaved cell lymphoma. Also called indolent lymphomas due to their slow growth.

The term "follicular B cell lymphoma" refers to a type of non- Hodgkin's lymphoma. It is an indolent (slow-growing) type of lymphoma.

Further definitions and characterizations of low-grade lymphomas can be found on the Internet at http://rituxan.com/professional/clinical_information/class/index.html.

The term "isolating" as refers to a protein or polypeptide, refers to removing a naturally occurring polypeptide or protein from its normal cellular environment or refers to removing a polypeptide or protein synthesized in an expression system (such as the baculovirus system described herein) from the other components of the expression system. Thus, the polypeptide sequence may be in a cell-free solution or placed in a different cellular environment. The term does not imply that the polypeptide sequence is the only amino acid chain present, but that it is essentially free (about 90 - 95% pure at least) of non-amino acid-based material naturally associated with it.

By the use of the term "enriched" in reference to a polypeptide is meant that the specific amino acid sequence constitutes a significantly higher fraction (2- to 5-fold) of the total amino acid sequences present in the cells or solution of interest than in normal or diseased cells or in the cells from which the sequence was taken. This could be caused by a person by preferential reduction in the amount of other amino acid sequences present, or by a preferential increase in the amount of the specific amino acid sequence of interest, or by a combination of the two. However, it should be noted that enriched does not imply that there are no other amino acid sequences present, just that the relative amount of the sequence of interest has been significantly increased. The term significant here is used to indicate that the level of increase is useful to the person making such an increase, and generally means an increase relative to other amino acid sequences of about at least 2-fold, more preferably at least 5- to 10-fold or even more. The term also does not imply that there is no amino acid sequence from other sources. The other source of amino acid sequences may, for example, comprise amino acid sequence encoded by a yeast or bacterial genome, or a cloning vector such as pUC19. In preferred embodiments, the amino acid sequence is a chimeric protein as described above. The term is meant to cover only those situations in which man has intervened to increase the proportion of the desired amino acid sequence.

It is also advantageous for some purposes that an amino acid sequence be in purified form. The term "purified" in reference to a polypeptide does not require absolute purity (such as a homogeneous preparation); instead, it represents an indication that the sequence is relatively purer than in the natural environment. Compared to the natural level this level should be at least 2-to 5-fold greater (*e.g.*, in terms of mg/mL). Purification of at least one order of magnitude, preferably two or three orders, and more preferably four or five orders of magnitude is expressly contemplated. The substance is preferably free of contamination at a functionally significant level, for example 90%, 95%, or 99% pure.

The term "operatively linked" refers to an arrangement of DNA in which a controlling region, such as a promoter or enhancer, is attached to a connected DNA gene of interest so as to bring about its transcription, and hence allowing its translation. The term "operatively linked" may also refer to a DNA sequence encoding a processing signal, such as a secretory signal sequence, connected to a gene encoding a polypeptide to form a single open reading frame. Following transcription and translation, the secretory signal sequence has the potential to bring about the export of the translated polypeptide. One skilled in the art is familiar with the meaning of this term.

### Functional Derivatives of Useful Chimeric Proteins

Also provided herein are functional derivatives of a polypeptide or nucleic acid of the invention. By "functional derivative" is meant a "chemical derivative," "fragment," or "variant," of the polypeptide or nucleic acid of the invention, as these terms are defined below. A functional derivative retains at least a portion of the function of the protein, for example, reactivity with an antibody specific for the protein or binding activity mediated through noncatalytic domains, which permits its utility in accordance with the present invention. It is well known in the art that due to the degeneracy of the genetic code numerous different nucleic acid sequences can code for the same amino acid sequence. Equally, it is also well known in the art that conservative changes in amino acid can be made to arrive at a protein or polypeptide that retains the functionality of the original. In both cases, all permutations are intended to be covered by this disclosure.

Included within the scope of this invention are the functional equivalents of the herein-described isolated nucleic acid molecules. The degeneracy of the genetic code permits substitution of certain codons by other codons that specify the same amino acid and hence would give rise to the same protein. The nucleic acid sequence can vary substantially since, with the exception of methionine and tryptophan, the known amino acids can be coded for by more than one codon. Thus, portions or all of the genes of the invention could be synthesized to give a nucleic acid sequence significantly different from a sequence that is found in nature. The encoded amino acid sequence thereof would, however, be preserved.

A "chemical derivative" of the complex contains additional chemical moieties not normally a part of the protein. Covalent modifications of the protein or peptides are included within the scope of this invention. Such modifications may be introduced into the molecule by reacting targeted amino acid residues of the peptide with an organic derivatizing agent that is capable of reacting with selected side chains or terminal residues, as described below. It may also consist of attaching carbohydrates to the protein in addition to the normal carbohydrates attached by the bacculovirus expression system of the invention.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluomacetone, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Parabromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect or reversing the charge of the lysinyl residues. Other suitable reagents for derivatizing primary amine containing residues include imidoesters such as methyl picolinimidate; pyridoxal phosphate; pyridoxal; chlorobomhydride; trinitrobenzenesulfonic acid; O-methylisourea; 2,4 pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine α-amino group.

Tyrosyl residues are well-known targets of modification for introduction of spectral labels by reaction with aromatic diazonium compounds or tetranitromethane. Most commonly, N-acetylimidizol and tetranitromethane are used to form O-acetyl tyrosyl species and 3-nitro derivatives, respectively.

Carboxyl side groups (aspartyl or glutamyl) are selectively modified by reaction with carbodiimide (R'-N-C-N-R') such as 1-cyclohexyl-3-(2-morpholinyl(4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Other modifications include the in vitro glycosylation of polypeptides or proteins.

Derivatization with bifunctional agents is useful, for example, for cross-linking the component peptides of the protein to each other or to other proteins in a complex to a water-insoluble support matrix or to other macromolecular carriers. Commonly used cross-linking agents include, for example, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[p-azidophenyl] dithiolpropioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include hydroxylation of proline and lysine, phosphorylation of hydroxyl groups of seryl or threonyl residues, methylation of the α-amino groups of lysine, arginine, and histidine side chains (Creighton, T.E., Proteins: Structure and Molecular Properties, W.H. Freeman & Co., San Francisco, pp. 79-86, 1983), acetylation of the N-terminal amine, and, in some instances, amidation of the C-terminal carboxyl groups.

Such derivatized moieties may improve the stability, solubility, absorption, biological half life, and the like. The moieties may alternatively eliminate or attenuate any undesirable side effect of the protein complex and the like. Moieties capable of mediating such effects are disclosed, for example, in Remington's Pharmaceutical Sciences, 18th ed., Mack Publishing Co., Easton, PA (1990).

A functional derivative of a protein with deleted, inserted and/or substituted amino acid residues may be prepared using standard techniques well-known to those of ordinary skill in the art. For example, the modified components of the functional derivatives may be produced using site-directed mutagenesis techniques (as exemplified by Adelman et al., DNA 2:183,1983) wherein nucleotides in the DNA coding the sequence are modified such that a modified coding sequence is modified, and thereafter expressing this recombinant DNA in a prokaryotic or eukaryotic host cell, using techniques such as those described above. Alternatively, proteins with amino acid deletions, insertions and/or substitutions may be conveniently prepared by direct chemical synthesis, using methods well-known in the art. The functional derivatives of the proteins typically exhibit the same qualitative biological activity as the native proteins.

### Uses of the Chimeric Proteins

An effective amount of chimeric protein is delivered according to the invention. "Effective amount" refers to an amount that results in the desired biological response being elicited. What constitutes such an amount will vary, and depends on a variety of factors, including the particular chimeric protein, the desired biological response to be elicited, the formulation of the chimeric protein, the age, weight, gender, and health of the organism to be treated, the dosage regimen, the condition or disease to be treated or prevented, etc.

The chimeric proteins described herein can be administered in pharmaceutical compositions where they are mixed with other active ingredients, as in combination therapy, or suitable carriers or excipient(s). Techniques for formulation and administration of the chimeric proteins may be found in "Remington's Pharmaceutical Sciences," Mack Publishing Co., Easton, PA, latest edition.

### 1. Routes of Administration.

Suitable routes of administration may, for example, include oral, rectal, transmucosal, or intestinal administration; parenteral delivery, including intramuscular, subcutaneous, intravenous, intramedullary injections, as well as intrathecal, direct intraventricular, intraperitoneal, intranasal, or intraocular injections. One of skill in the art will understand the various modifications that would be made to adapt the composition to a particular route of administration.

### 2. Composition/Formation.

The pharmaceutical compositions may be manufactured in a manner that is itself knows, *e.g.*, by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the agents of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Suitable carriers include excipients such as, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.*, gelatin for use in an inhaler or insufflator may be formulated containing a powder mix of the compound and a suitable powder base such as lactose or starch.

The compounds may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, *e.g.,* sterile pyrogen-free water, before use.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example, as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:D5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied, other biocompatible polymers may replace polyethylene glycol, *e.g*., polyvinyl pyrrolidone; and other sugars or polysaccharides may substitute for dextrose.

Alternatively, other delivery systems for compositions may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the compounds of the invention may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents that are the corresponding free base forms.

### 3. Effective Dosage.

Pharmaceutical compositions suitable for use in the present invention include compositions where the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

Toxicity and therapeutic efficacy of the compounds described herein can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See *e.g*., Fingl et al., "The Pharmacological Basis of Therapeutics," Ch. 1 p.1, 1975).

Dosage amount and interval may be adjusted individually to provide plasma levels of the active moiety which are sufficient to maintain the required effect, or minimal effective concentration (MEC). The MEC will vary for each compound. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

### 4. Packaging.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the polynucleotide for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition. Suitable conditions indicated on the label may include treatment of a tumor, treatment of rheumatoid arthritis, treatment of diabetes, and the like.

### EXPERIMENTAL SECTION

### 1. TISSUE PROCESSING FOR NON-HODGKIN'S LYMPHOMA IDIOTYPE (ID) IDENTIFICATION AND CLONING:

Tumor samples from a peripheral lymph node were biopsied as clinically indicated under sterile conditions and used to generate patient idiotype-specific recombinant chimeric immunoglobulin proteins. Remaining lymph node biopsy material was stored in liquid nitrogen in tissue cell bank for future use.
a. Cell Isolation: Single cell suspensions of patient lymph node biopsies were obtained by forcing the biopsied lymphoma tissue through a disposable 0.38 mm steel mesh screen while submerged in sterile PBS. The dispersed cells were washed twice in PBS, then resuspended and counted. A 10% fraction of the cells were processed for total RNA extraction and the remaining cells were archived in liquid nitrogen following resuspension in RPMI 1640 tissue culture media containing 30% (v/v) fetal bovine serum and 10% (v/v) DMSO. All processing of clinical samples was performed in a biological safety cabinet.
b. Total RNA Preparation: Total RNA from homogenized lymph node cells was isolated using RNeasy Kit (Qiagen) as per manufacturer's instruction. Total RNA was quantitated by spectrophotometry.
c. cDNA Synthesis: Approximately 2.0 µg total RNA was used as template for first strand cDNA synthesis using the SuperScript Preamplification System (GIBCO-BRL) according to manufacturer's recommendation. Oligo (dT) provided with the kit was used to prime the cDNA.
d. PCR Amplification of Genes Encodine Lymphoma Heavy and Light Chains: Both heavy and light chains from the lymphoma-specific immunoglobulins were identified as follows. Aliquots of the single stranded lymphoma cDNA were combined with a series of V_{H} and V_{L} leader sequence-specific oligonucleotide sense primers representing all known V_{H}, V_{κ}, and V_{λ} subfamilies as listed in Table 1, paired with IgM, IgG, IgA, or Ig_{κ} and Ig_{λ} constant region specific antisense primers. These samples were then amplified by PCR and analyzed by agarose gel electrophoresis.
Parallel reactions were conducted using cDNA prepared from the patient's peripheral blood lymphocytes. A comparison ofPCR products generated by each pair of primers derived from samples containing normal PBL or lymph node biopsy cDNA would lead to the identification of the candidate tumor specific V_{H} and V_{κ} or V_{λ} subfamily over-represented in the lymphoma, and the isotype of the heavy and light chains. Candidate tumor V region gene products were then excised, and their nucleic acid sequence was determined to assess clonality. For each patient, two independent analyses were performed from starting cellular fractions.
One microliter of the cDNA reaction (representing 5% of the total cDNA reaction volume) was amplified for 35 cycles in 50 µl volume using the HotStarTaq Master Mix Kit (Qiagen). Cycling conditions: 95 °C 15 min, 65 °C 4 min, 72 °C 1 min, followed by 94 °C 1 min, 61 °C 30 sec, 72 °C 1 min for 34 cycles; and a final extension step at 72 °C for 7 min. A 10 µl aliquot of each reaction is analyzed by electrophoresis on a 1% agarose gel with ethidium bromide.

| **TABLE 1.** | **Primer Sequences Used for Amplification of Lymphoma Heavy and Light Chains** | |
|---|---|---|
| '(GA)' means either a G or an A, '(TC)' means either a T or a C. | | |
| | PRIMER NAME | PRIMER SEQUENCE (5' 3') |
| 491 | V_{H1}L | TCACCATGGACTGGACCTGGAG SEQ ID NO:38 |
| 492 | V_{H2}L.1 | ACCATGGACATACTTTGTTCCACGC SEQ ID NO:39 |
| 493 | V_{H2}L.2 | ACCATGGACACACTTTGCTCCACGC SEQ ID NO:40 |
| 494 | V_{H3}L.1 | ACCATGGAGTTTGGGCTGAGCTG SEQ ID NO:41 |
| 495 | V_{H3}L.2 | ACCATGGAACTGGGGCTCCGCTG SEQ ID NO:42 |
| 496 | V_{H4}L | AAGAACATGAAACACCTGTGGTTCTTC SEQ ID NO:43 |
| 497 | V_{H5}L | ATCATGGGGTCAACCGCCATCCT SEQ ID NO:44 |
| 498 | V_{H6}L | ACAATGTCTGTCTCCTTCCTCATC SEQ ID NO:45 |
| 516 | V_{κ1}L | ACATGAGGGTCCCCGCTCAGC SEQ ID NO:46 |
| 517 | V_{κ2}L | TCAGCTCCTGGGGCTGCTAATG SEQ ID NO:47 |
| 515 | V_{κ3}L | CTTCCTCCTGCTACTCTGGCTC SEQ ID NO:48 |
| 518 | V_{κ4}L | GCAGACCCAGGTCTTCATTTCTC SEQ ID NO:49 |
| 519 | V_{κ5}L | CCAGGTTCACCTCCTCAGCTTC SEQ ID NO:50 |
| 520 | V_{κ6}L | GGTTTCTGCTGCTCTGGGTTCC SEQ ID NO:51 |
| 522 | V_{λ1}L | TCACTG(TC)(GA)CAGGGTCCTGGGC SEQ ID NO:52 |
| 523 | V_{λ2}L | ACTCAGG(GA)CACAGG(GA)TCCTGG SEQ ID NO:53 |
| 524 | V_{λ3}L.1 | TTGCTTACTGCACAGGATCCGTG SEQ ID NO:54 |
| 525 | V_{λ3}L.2 | CTTGCTCACTTTACAGGTTCTGTG SEQ ID NO:55 |
| 526 | V_{λ3}L.3 | CTCACTCTTTGCATAGGTTCTGTG SEQ ID NO:56 |
| 527 | V_{λ3}L.4 | TCAACCTCTACACAGGCTCTATTG SEQ ID NO:57 |
| 528 | V_{λ3}L.5 | CTCACTCTCTGCACAG(GT)CTCTG(AT)G SEQ ID NO:58 |
| 529 | V_{λ4}.L1 | CATTTTCTCCACAGGTCTCTGTGC SEQ ID NO:59 |
| 530 | V_{λ4}L.2 | CCTCCACTG(GC)ACAGGGTCTCTC SEQ ID NO:60 |
| 531 | V_{λ5}L | CTCTCACTGCACAGGTTCCCTC SEQ ID NO:61 |
| 532 | V_{λ6}L | CGCTCACTGCACAGGTTCTTGG SEQ ID NO:62 |
| 533 | V_{λ7}L | CTTGCTGCCCAGGGTCCAATTC SEQ ID NO:63 |
| 534 | V_{λ8}L | TGCTTATGGATCAGGAGTGGATTC SEQ ID NO:64 |
| 535 | V_{λ9}L | CAGTCTCCTCACAGGGTCCCTC SEQ ID NO:65 |
| 536 | V_{λ10}L | TCACTCACTCTGCAGTGTCAGTG SEQ ID NO:66 |
| | IgG Constant-E | CTGAGTTCCACGACACCGTCAC SEQ ID NO:69 |
| | IgM Constant-E | GGGAATTCTCACAGGAGACGAGG SEQ ID NO:70 |
| | C_{κ}-E | TTGGAGGGCGTTATCCACCTTC SEQ ID NO:71 |
| | C_{λ}-E | GAAGTCACTTATGAGACACACCAG SEQ ID NO:72 |
| | IgG Constant- I | GGAAGTAGTCCTTGACCAGGCAG SEQ ID NO:73 |
| | IgM Constant- I | GGGAAAAGGGTTGGGCCCGATGCAC SEQ ID NO:74 |
| | C_{κ}-I | GGGAAAAGGGTTGGGCCCGATGCAC SEQ ID NO:75 |
| | C_{λ}-I | GGAACAGAGTGACACTGGGTGCAGCCTTGGGCTG SEO ID NO:76 |
| | Cλ Downstream | TGCCGTCGGCAGGAGGTATTTCATTATGACTGTCT CCTTGCTATTATGAACATTCTGTAGGGGCCA SEQ ID NO:77 |
| | Cλ - 5' | GTCAGCCCAAGGCTGCACCCAGTGTCACTCTGTTCC SEQ ID NO:78 |
| | Cλ - 3' | CGTATCAAGCTTTTACTATGAACATTCTGTAGGGGC CAC SEQ ID NO:79 |
| | λ-stuff 1 | CCTTTGATAACACCCA SEQ ID NO:80 |
| | λ-stuff 1' | GTGTTATCAAAGG SEQ ID NO:81 |
| | γ1-stuff 1 | 5'-CTAGTTTGATAAGGGCC-3' SEQ ID NO:82 |
| | γ1-stuff 1' | 5'-CTTATCAAA-3' SEQ ID NO:83 |
| | κ-stuff 1 | 5'-CCTTTGATAACACCAA-3' SEQ ID NO:84 |
| | κ-stuff 1' | 5'- -3' SEQ ID NO:85 |

e. Cloning and Sequencing of PCR Products: PCR products from reactions determined to contain the tumor specific variable sequences for heavy and light chains were cloned directly into plasmid pCR2.1-TOPO as per manufacturer's recommendations, and introduced into Top10 competent *E. coli* cells (Invitrogen). Twenty four miniprep DNA plasmids were prepared from carbenicillin resistant bacterial colonies using the QIAprep Spin Miniprep Kit (Qiagen), and quantitated by spectrophotometry. Two hundred ng of each plasmid was sequenced using the Cy5/Cy5.5 Dye Primer Cycle Sequencing Kit (Visible Genetics). Following the completion of the sequencing reactions, samples were electrophoresed on the OpenGene Automated DNA Sequencing System and the data was processed with GeneObjects software package (Visible Genetics). Additional analysis including sequence alignments were performed using the SEQUENCHER Version 4.1.2 DNA analysis software (GENE Codes Corp.). A V-region derived sequence could be considered tumor specific if it was present in 75% of the samples, for example, if 18 or greater of the 24 form a consensus group when analyzed using the above software utilizing the default parameters. Two independent biopsy samples would be compared when available.
f. cDNA Synthesis and Generation of 5'RACE Products: Due to the occurrence of mutations in the V_{H} and V_{L} sequences, it is not possible at times to identify tumor-specific immunoglobulin rearrangements. As an alternative to the sequence-specific PCR strategy *supra,* one can employ a 5' RACE PCR strategy to identify tumor specific immunoglobulin (Ig) rearrangements. All steps for first strand cDNA synthesis to the generation of Ig specific PCR products are performed according to manufacturer's directions (5' RACE system for Rapid Amplification of cDNA Ends, version 2.0, Gibco BRL), with slight modification. Approximately 2.5 µg of total RNA is used as template for each first strand cDNA synthesis in the presence of specific antisense primers complimentary to the immunoglobulin heavy and the light chains' constant region utilized by the B cell population of interest (SEQ ID NO:69 for IgG, SEQ ID NO:70 for IgM, SEQ ID NO:71 for C_{κ}, and SEQ ID NO:72 for C_{λ}). The cDNA reactions are purified over GlassMAX spin cartridges, generating a final volume of 50 µl each. A 10 µl aliquot of each purified cDNA is oligo(dC) tailed with terminal deoxynucleotidyl transferase in a 25 µl volume, generating the templates to be used for subsequent PCR reactions. The PCR set up utilizes an upstream primer containing a poly(G) track provided by the manufacturer and an Ig specific antisense primer interior to that used for cDNA first strand synthesis (SEQ ID NO:73 for IgG, SEQ ID NO:74 for IgM, SEQ ID NO:75 for C_{κ}, and SEQ ID NO:76 for C_{λ}). Five µl of template is amplified in a 50 µl volume as follows: 95 °C for 15 min, 55 °C for 4 min, 72 °C for 1 min, followed by 94 °C for 1 min, 55 °C for 30 sec, 72 °C for 1 min for 34 cycles, and a final extension step at 72 °C for 7 min. The final PCR products are separated by electrophoresis on a 1% agarose gel with ethidium bromide and the band of the appropriate size (~500-600 bp) is isolated are cloned into the pCR2.1-TOPO plasmid as described in 1e, *supra.*

### 2. CONSTRUCTION OF BACULOVIRUS EXPRESSION VECTORS pTRABacHuLC_{κ}HC_{γ1} AND pTRABacHuLC_{λ}HC_{γ1}

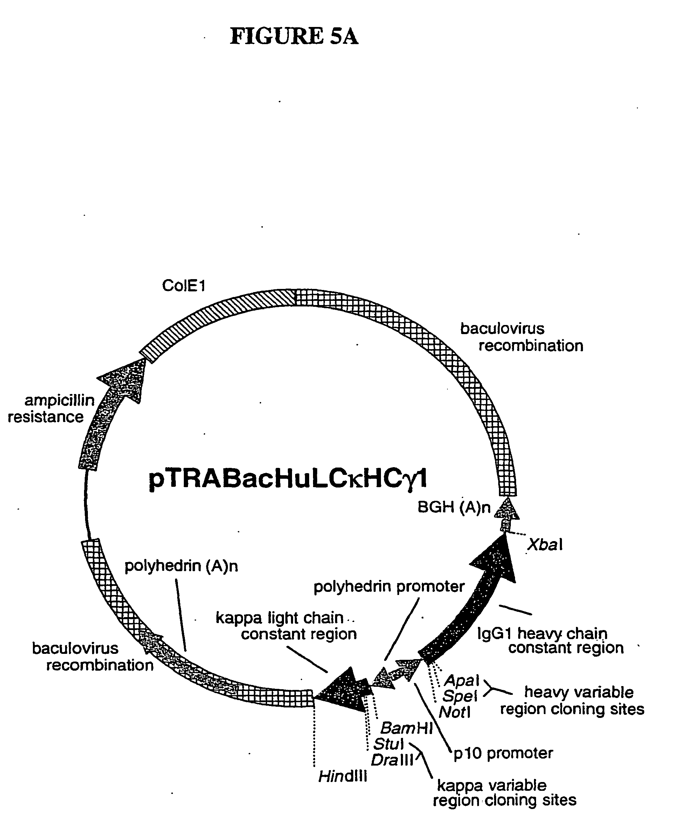

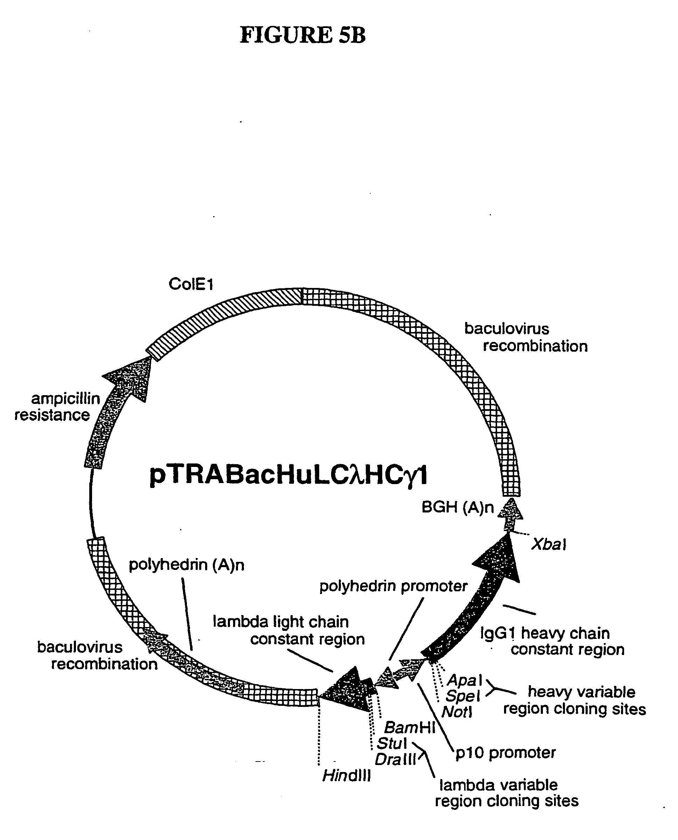

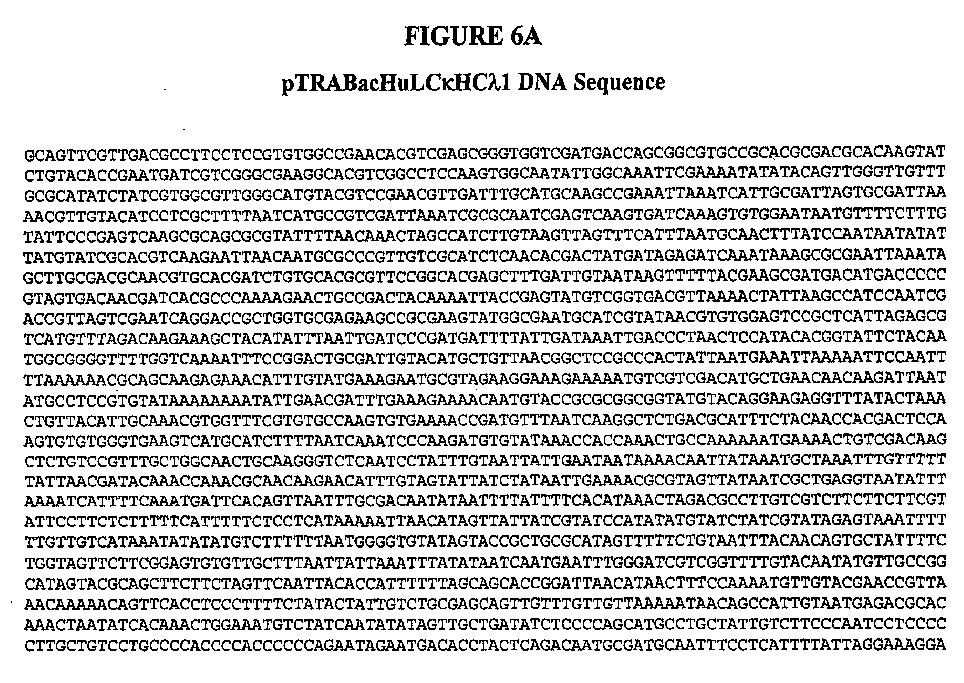

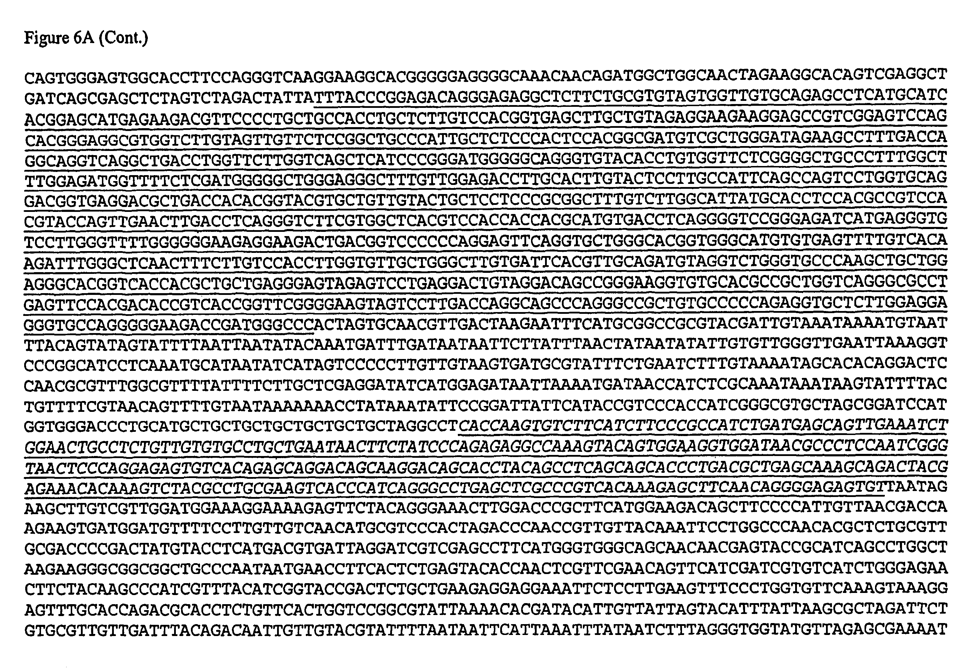

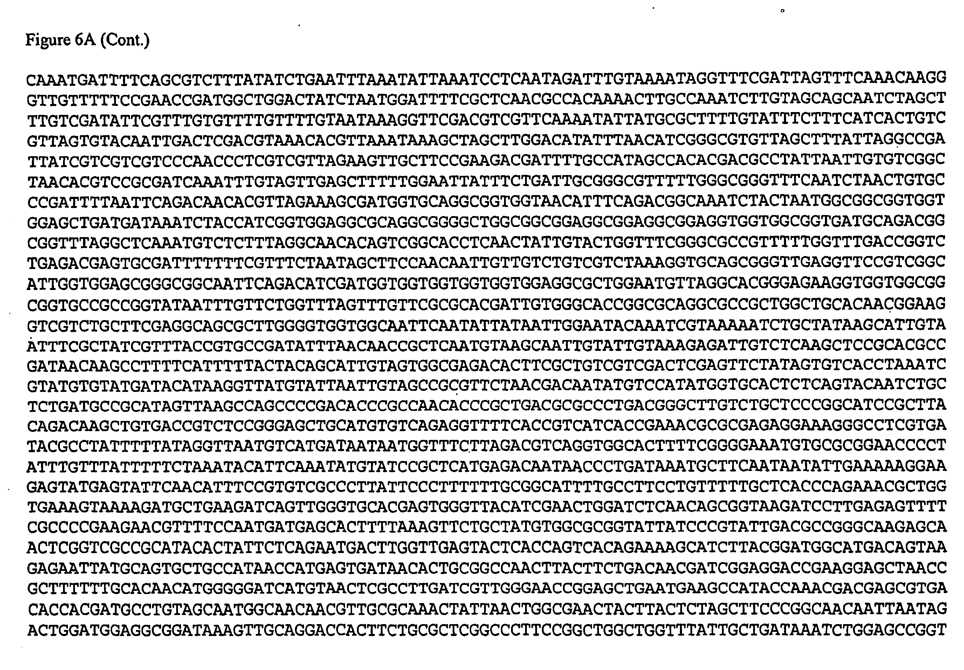

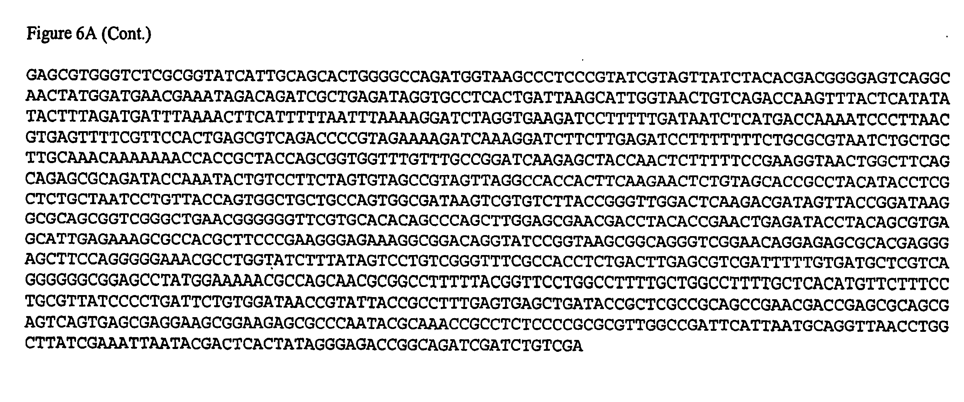

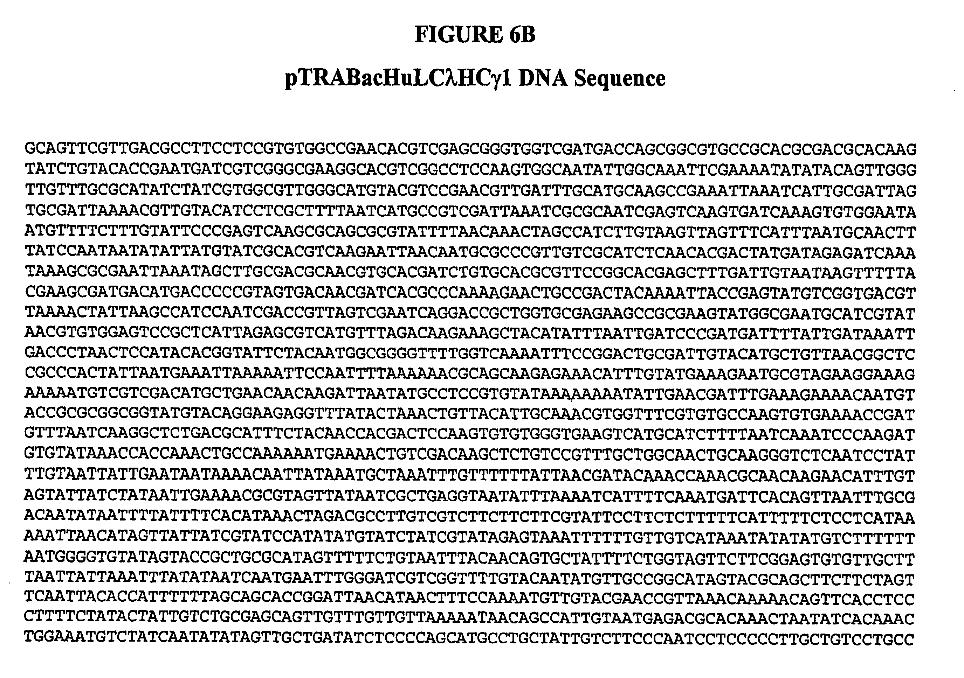

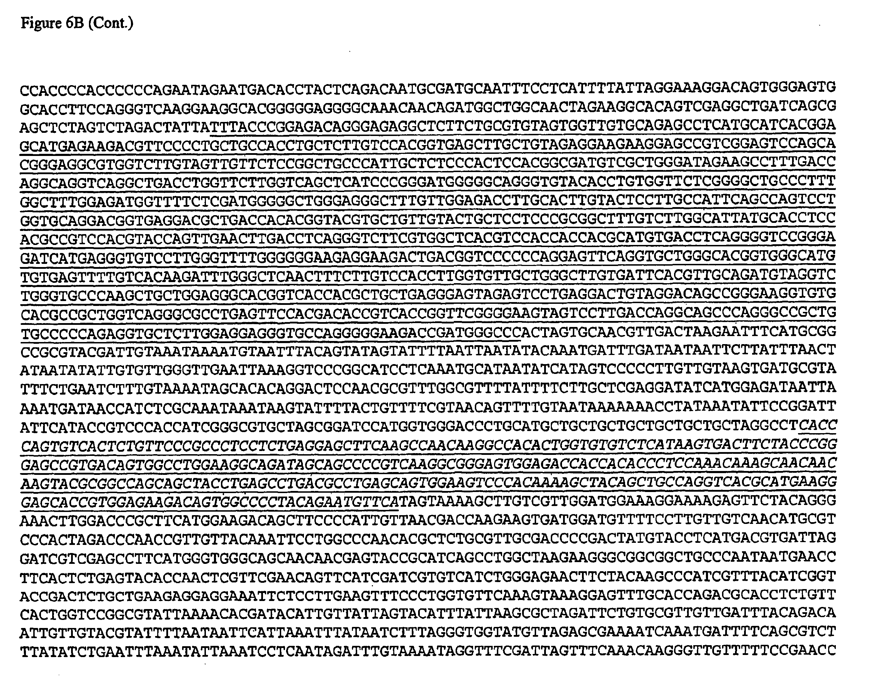

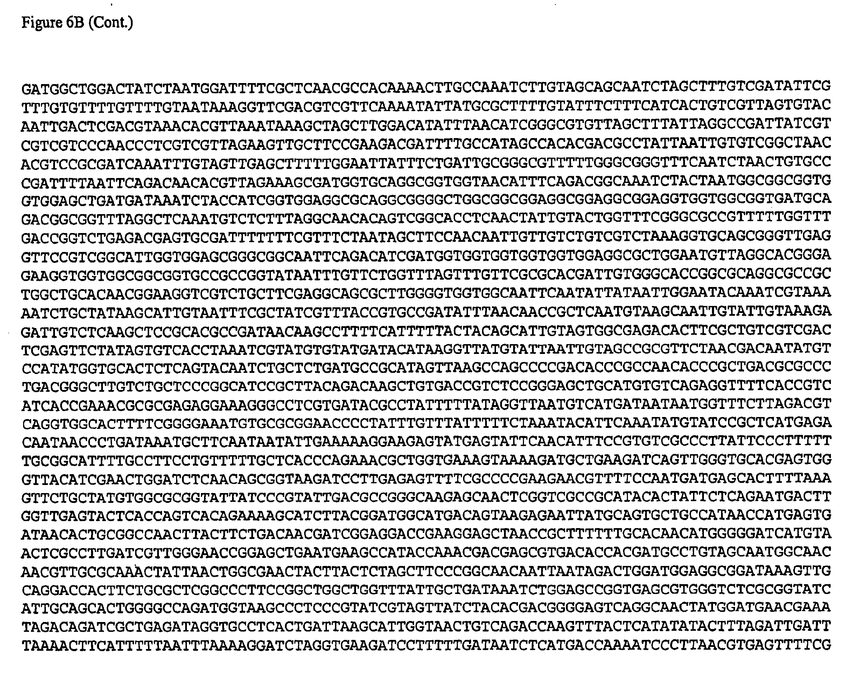

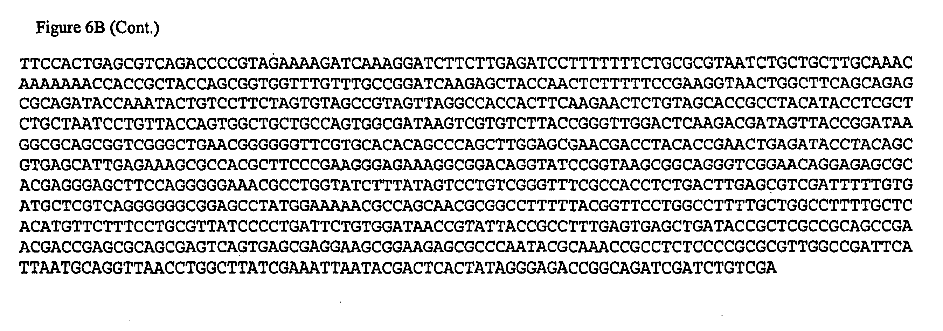

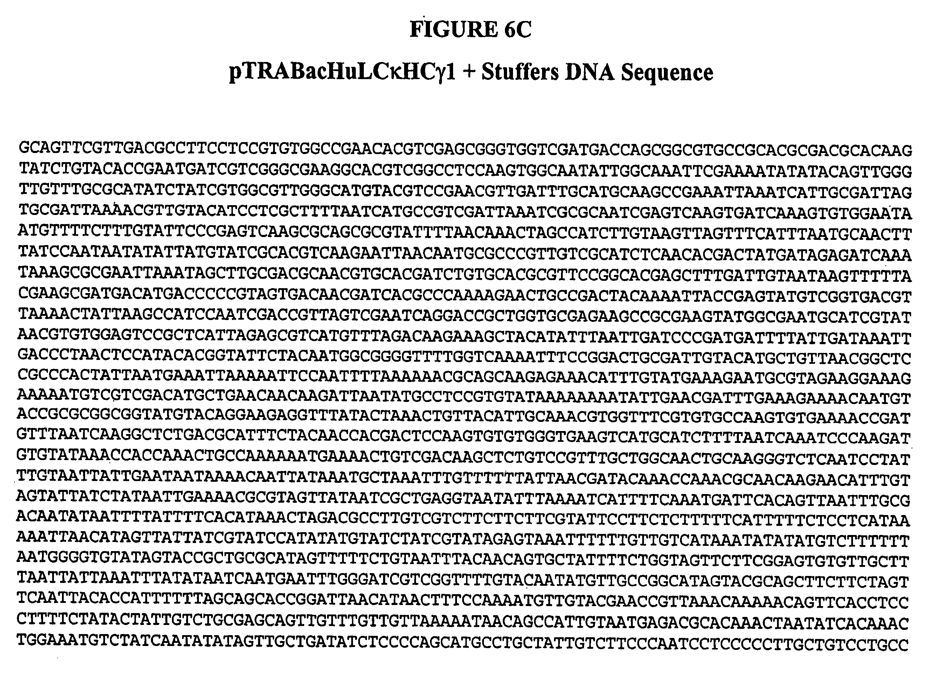

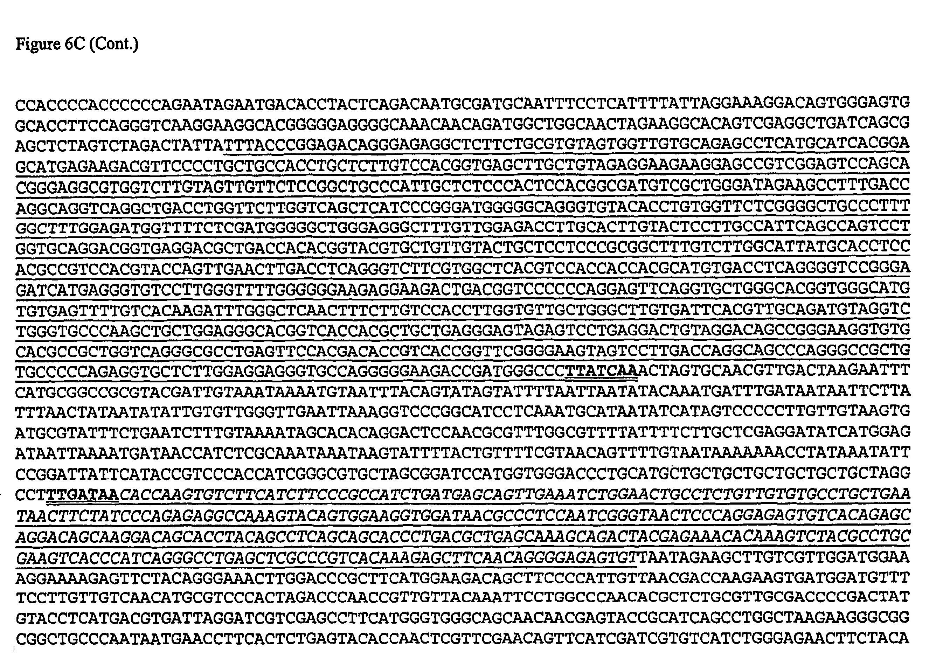

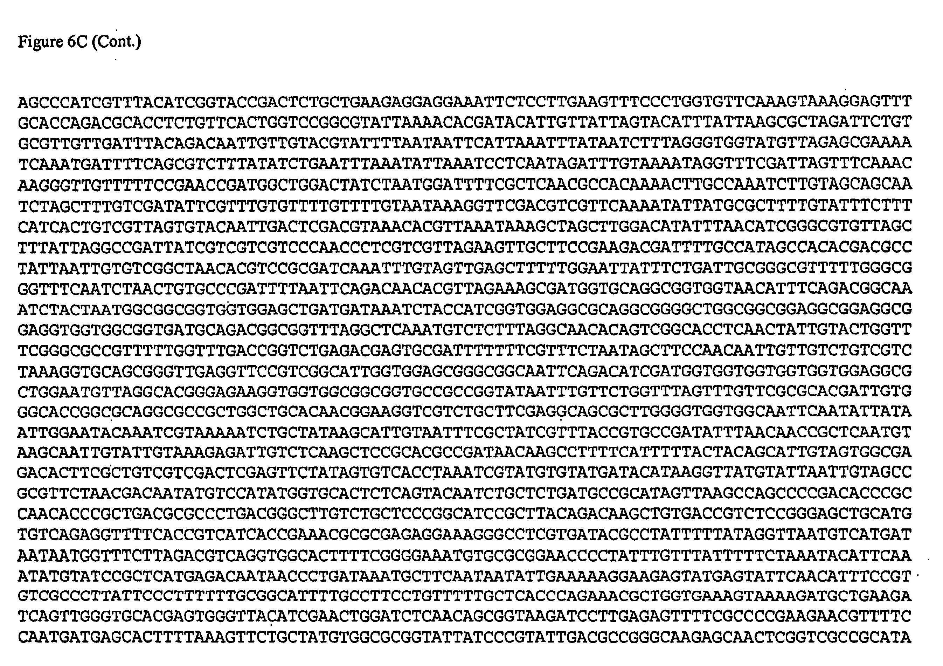

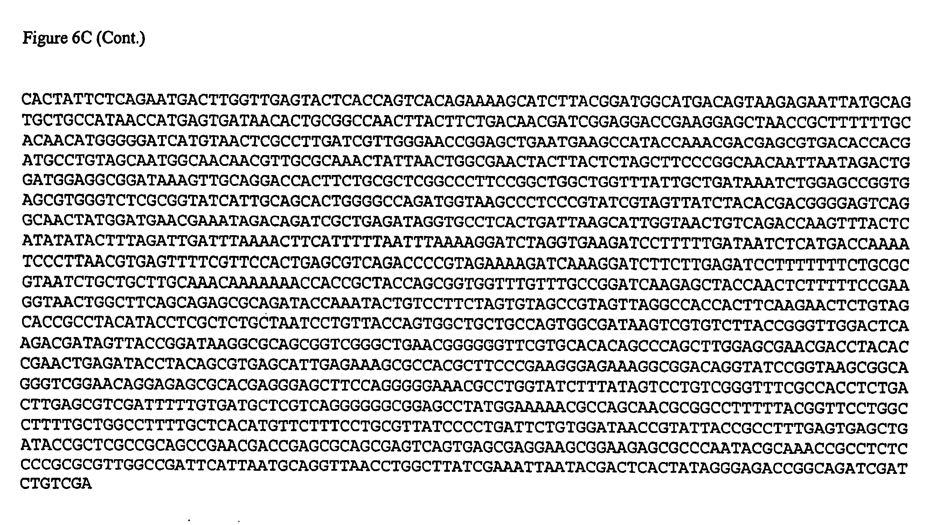

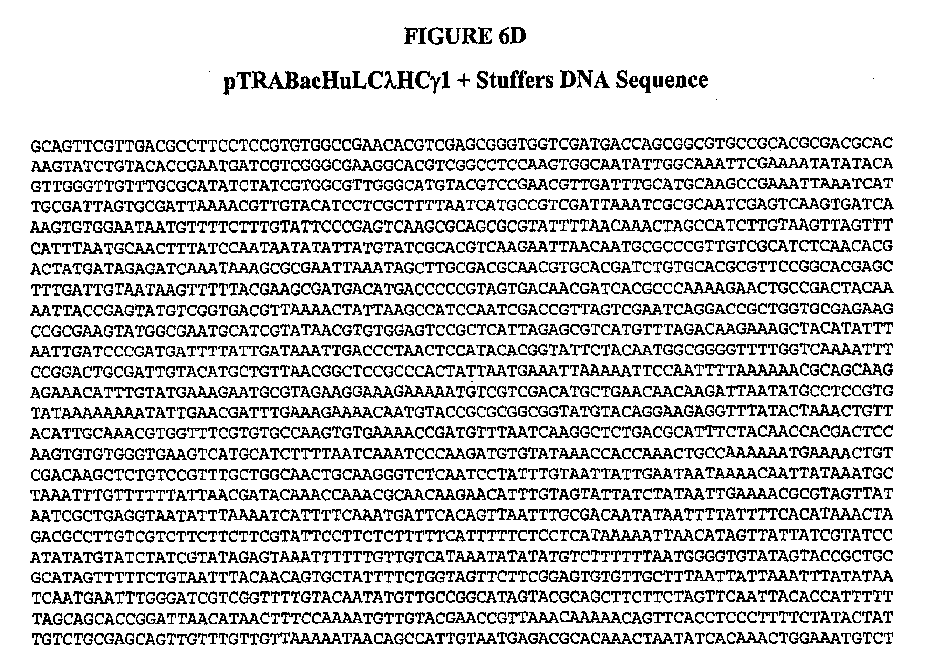

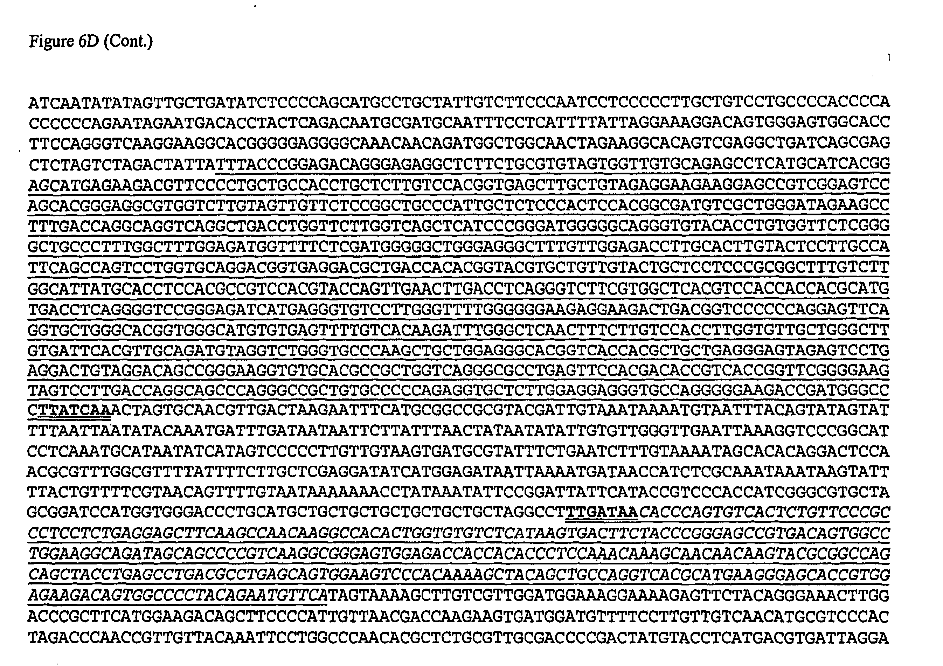

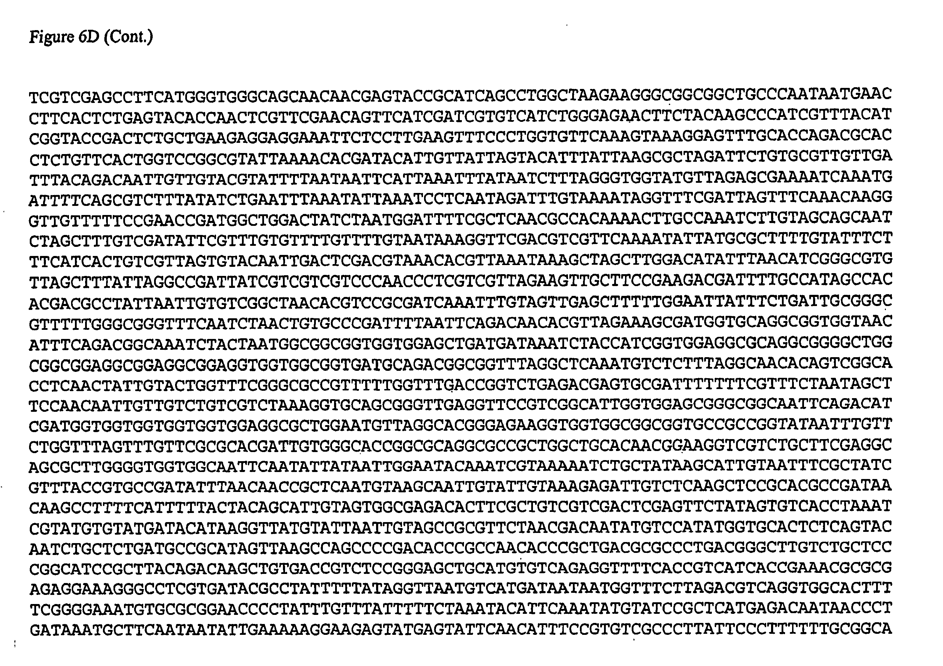

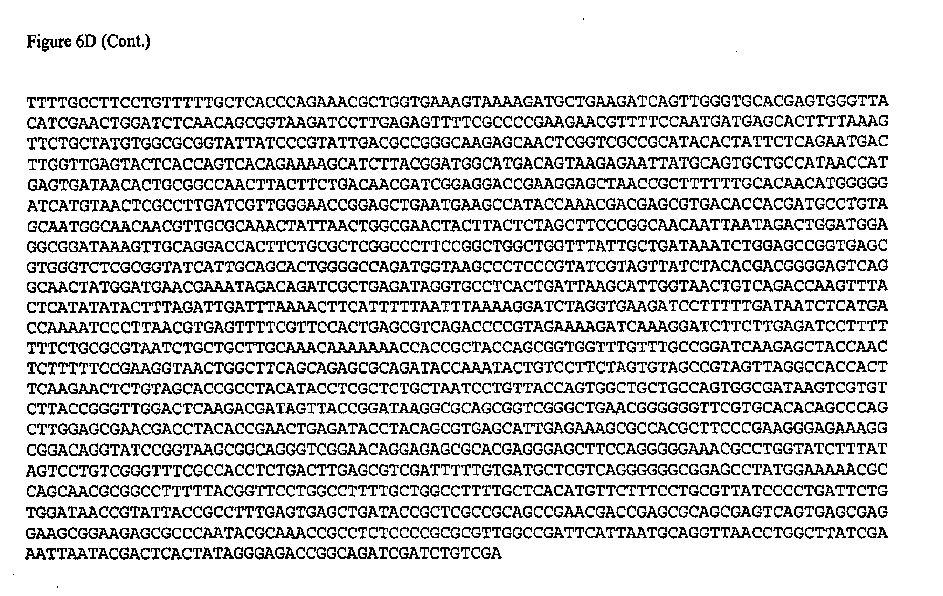

a. Cloning of Secretory Signal Sequences into p2Bac: The base vector for the pTRABacHuLC_{κ}HC_{γ1} and pTRABacHuLC_{λ}HC_{γ1} constructs was p2Bac (Fig. 2, SEQ ID NO:5, Invitrogen, Carlsbad, CA). Two secretory signal sequences were cloned into this base vector, and the first intermediate baculovirus expression vector p2BacM was created. In general, the vector p2Bac was first modified utilizing complimentary oligonucleotides encoding the amino terminal domain of the honey bee melittin secretory signal sequence positioned to be under transcriptional control of the baculoviral AcNPV P10 promoter. For melittin sequence cloning, 2 µg p2Bac was digested with Not I and Spe I for 4 hours at 37 °C. The linear vector was purified following electrophoresis through a 1% agarose gel using Qiaex II resin (Qiagen, Chatsworth, CA). The purified DNA was then eluted with 50 µl water and the DNA concentration was determined. One µg each of primers Me1S/N (SEQ ID NO:15) and MelN/S (SEQ ID NO:16) were mixed in 10 µl digestion buffer M (Roche Molecular Biochemicals, Indianapolis, IN), and heated to 70 °C for 5 min, then cooled to room temperature to anneal complimentary primers. Ten percent of the annealed primers was digested in 20 µl reaction with Not I and Spe I for 4 hours at 37 °C, and the digested primers were purified following electrophoresis through a 15% polyacrylamide gel with Qiaex H resin, and the concentration of the DNA for annealed primers was determined. The DNAs of p2Bac vector and annealed melittin fragment were ligated at 1:10 vector to insert ratio. The ligation product was transformed using competent XL1-Blue *E*. *coli* (Stratagene, San Diego, CA) and plated on a LB-carbenicillin agar plate for overnight growing at 37 °C. Miniprep colonies were prepared by standard protocols, and the plasmids were sequenced to check the construction. The resulting vector p2BacM contained the melittin secretory signal sequence.
   The p2BacM vector was further modified similarly to encode for the amino terminal domain of the human placental alkaline phosphatase secretory signal sequence under transcriptional control of the AcNPV polyhedron promoter, creating a second intermediate baculovirus expression vector p2BacMA. The procedure used to introduce the alkaline phosphatase sequence was generally cloned as follows: 2 µg p2BacM plasmid was digested with Bam HI and Eco RI, the linear vector was gel purified from agarose gel with Qiaex II resin and eluted in 50 µl water. The DNA concentration of the vector was determined. One µg each of primers APB/E (SEQ ID NO:17) and APE/B (SEQ ID NO:18) were mixed in 10 µl digestion buffer M, and heated to 70 °C for 5 min and then cooled down to room temperature to anneal complimentary primers. Ten percent of the annealed primers was digested in a 20 µl reaction with Bam HI and Eco RI for 4 hours at 37 °C. The digested primers were then purified from 15% polyacrylamide gel with Qiaex II resin. The DNA concentration of the digested primers was also determined. The linear p2BacM vector and alkaline phosphatase fragment were then ligated at 1:10 vector to insert ratio, and the ligation product was transformed using competent XL1-Blue *E. coli* and plated on a LB-carbenicillin agar plate for overnight growing at 37 °C. Miniprep colonies were prepared and the plasmids were sequenced to check the construction. The resulting intermediate vector p2BacMA would contain a secretory signal sequence for a human placental alkaline phosphatase. The p2BacMA plasmid was further transformed into SCS-110 *E*. *coli* strain (Stratagene) lacking *dcm* methylase activity for subsequent cloning of the κ constant region into methyl-sensitive Stu I site.
b. Amplification and Cloning of Constant Regions of IgG_{γ1} and Light Chains: The human kappa (κ) constant and the human IgG_{γ1} constant domains of human monoclonal antibody 9F12 were PCR amplified from RNA extracted from the human cell line 9F12 (ATCC#HB8177). The κ constant region was cloned behind the alkaline phosphatase signal sequence. The IgG_{γ1} constant region was inserted downstream from the melittin secretory signal sequence thus creating the vector (pTRABacHuLC_{κ}HC_{γ1}, Fig. 5a). A vector containing the human lambda (λ) light chain constant region (pTRABacHuLC_{λ}HC_{γ1}, Fig. 5b) was produced by replacing the κ light chain constant region with a λ light chain constant region. The light chains were isolated by RT-PCR from a chronic lymphocytic leukemia cellular RNA preparation. The detailed description of the cloning procedures are as follows.
c. Amplification of 9F12 κ and IgG_{γ1} constant region fragments: Total RNA from 9FI2 cells (ATCC#BB8177) was extracted using the RNeasy Kit (Qiagen) as per the manufacturer's instruction. A single stranded cDNA was synthesized using SuperScript reverse transcriptase (GIBCO BRL, Rockville, MD) with oligo(dT) primers. One twentieth of the synthesized single strand cDNA was amplified in 100 µl PCR reactions with Expand High Fidelity Taq (Roche) using κ and IgG_{γ1} specific oligonucleotides (SEQ ID NO:21 plus SEQ ID NO:22 and SEQ ID NO:19 plus SEQ ID NO:20, respectively). The fragments from amplified 9FI2 immunoglobulin were purified from 1.5% SeaKem agarose with Qiaex II resin and eluted with 50 µl water. The DNA concentrations for the fragments were determined. The purified 9F12 immunoglobulin fragments were ligated separately into the TA-II (Invitrogen) PCR cloning vector. The ligation products were transformed using competent XLI-Blue *E. coli* and plated on a LB-carbenicillin agar plate for overnight growing at 37 °C. Miniprep colonies were prepared and the plasmid DNA was sequenced.
d. Insertion of the 9F12 κ Constant Region into the Expression Vector: For κ constant domain, 5 µg plasmid DNA containing a κ constant region and 2 µg of DNA for the vector p2BacMA purified from SCS110 *E. coli* were digested with Stu I and Hind III. A 320 bp fragment containing κ constant region and a 7.1 kb fragment containing p2BacMA vector were gel purified with Quiex II and eluted in 50 µl water. The DNA concentrations for both fragments were determined. The purified fragments were then ligated with Rapid Ligation Kit (Roche). The ligation products were transformed using competent XL1-Blue *E*. *coli* and plated on a LB-carbenicillin agar plate for overnight growing at 37 °C. Miniprep bacterial colonies were prepared and the recombinant DNA was sequenced to verify proper κ constant region insertion. The resulting plasmid vector was pTRABacLC_{κ}.
e. Addition of the IgG_{γ1} Constant Domain to the Vector: The IgG_{γ1} constant domain was added to the vector by first digesting 5 µg of plasmid DNA containing IgG_{γ1} constant region and 2 µg plasmid DNA for the vector pTRABacLC_{κ} with Spe I and Xba L A 1 kb fragment of IgG_{γ1} constant region and a 7.4 kb fragment of pTRABacLC_{κ} vector were gel purified from agarose plugs with Quiex II and eluted in 50 µl water. The DNA concentrations for both fragments were determined. The purified fragments were then ligated with Rapid Ligation Kit (Roche). The ligation products were transformed using competent XL1-Blue *E*. *coli* and plated on a LB-carbenicillin agar plate for overnight growing at 37 °C. Miniprep colonies were prepared and the ligation and orientation of the IgG_{γ1} insertion were determined by restriction analysis and sequencing of the restriction sites. The resulting recombinant vector was pTRABacHuLC_{κ}HC_{γ1}.
   This plasmid, pTRABacHuLC_{κ}H_{γ1}, was further refined to add translational stop codons between the melittin secretory sequence, and the C_{γ1} region sequence and the alkaline phosphatase secretory sequence and the C_{κ} region sequence, respectively. To accomplish these modifications, the pTRABacHuLC_{κ}HC_{γ1} vector was linearized following digestion with Spe I + Apa I. The linearized vector was then ligated with annealed complimentary primers γ1-stuff 1 (SEQ ID NO:82) and γ1-stuff 1' (SEQ ID NO:83) to introduce the in-frame stop codons. The vector resulting from this modification was subsequently linearized following digestion with Stu I (AGGCCT) + Dra III (CACnnnGTG) and then ligated with annealed complimentary primers κ-stuff 1 (SEQ ID No. 84) and _{κ}-stuff 1' (SEQ ID NO:81) to introduce the in-frame stop codons. The net effect of these modifications are indicated in the sequences shown in Figures 6C & 6D, respectively. (The added sequences are highlighted by a double underline and bold.)
f. Addition of the λ Constant Region to the Vectors:̅ T̅otal RNA from purified peripheral blood lymphocytes (PBL) obtained from a chronic lymphocytic leukemia (CLL) patient displaying a λ light chain idiotype was extracted using the RNeasy kit (Qiagen).

Approximately 2.0 µg total RNA was used as template for first strand cDNA synthesis using the SuperScript Preamplification System (Gibco BRL) according to manufacturer's recommendation. Oligo(dT) was used for priming. One twentieth of the synthesized single stranded cDNA was amplified in a PCR reaction using an upstream primer identical to a portion of the Vλ signal sequence (SEQ ID NO:54) and a downstream primer (SEQ ID NO:77) complimentary to the last several codons of the λ constant region as well as a portion of the 3' untranslated region. The PCR products were cloned into the pCRII vector (Invitrogen) and sequenced to confirm identity. A plasmid containing the correct λ constant region sequence was chosen as a template for a second PCR. In this reaction a sense oligonucleotide, Cλ-5' (SEQ ID NO:78), containing an engineered Dra III restriction site, corresponding the sequence in the λ constant region immediately downstream of Jλ and a Hind III containing antisense oligonucleotide primer, Cλ-3' (SEQ ID NO:79) spanning the STOP codon immediately following the λ constant region were utilized. The resulting PCR product was cloned into the pCR2.1-TOPO vector and sequenced. A fragment containing the λ constant region sequence was released upon Hind III restriction from some of the plasmids, depending on orientation of the insert. This restriction fragment was gel isolated and cloned into pTRABacHuLCκHCγ1 (Figure 5A), following linearization following Hind III digestion, generating an intermediate plasmid containing both the λ and κ constant regions. Restriction of this plasmid with Stu I and Dra III resulted in the removal of the κ sequences. This linearized plasmid was then ligated with annealed complimentary primers λ-stuff 1 (SEQ ID NO:80) and λ-stuff 1' to generate the final version of pTRABacHuLCλHCγ1 (Figure 5B).

### 3. INSERTION OF GENES FOR PATIENT-DERIVED IDIOTYPE V_{H} ND/OR V_{L} REGIONS INTO AN EXPRESSION VECTOR

Using either pTRABacHuLC_{κ}HC_{γ1} or pTRABacHuLC_{λ}HC_{γ1}, it was possible to insert genes for any V_{L} region containing the unique cloning sequences Stu I and Dra III between the alkaline phosphatase signal sequence and the κ or λ constant region, and genes for any V_{H} region containing the unique cloning sequences *Spe* I and *Apa* I between the melittin secretory signal sequence and the IgG_{γ1} constant region (See Figure 5A and 5B). The resulting expression vector could then be utilized for transduction into *Spodoptera frugiperda* (Sf-9) insect cells to produce recombinant budded baculovirus. The recombinant baculovirus was then serially amplified in Sf-9 cells to produce a high titer recombinant baculovirus stock. This high titer recombinant baculovirus stock was then used to infect *Trichoplusia ni* (High-5) cells for subsequent chimeric IgG protein production. A list of all oligonucleotide primers used in the construction of pTRABacHuLC_{κ}HC_{γ1} or pTRABacHuLC_{λ}HC_{γ1} can be found in Table 2.

After the tumor derived sequences for V_{H} and/or V_{L} regions are isolated as described above, oligonucleotide primers including the terminal 40 nucleotides of the melittin leader peptide (for Ig heavy chain cloning) (SEQ ID NO:8 - CAGATCACTA GTTTTTATGG TCGTGTACAT TTCTTACATC TATGCG], the terminal 28 nucleotides of the alkaline phosphatase leader peptide (for Ig light chain cloning) (SEQ ID NO:9 - CTGAGTAGGC CTGAGGCTAC AGCTCTCCCT GGGC), and the first 21 to 24 nucleotides of the respective V_{H} or V_{L} region proteins are prepared. Reverse oligonucleotide primers from the heavy or light chain constant region are used (IgG: SEQ ID NO:10 - GGAAGTAGTC CTTGACCAGG CAG; IgM: SEQ ID NO:11-GGGAAAAGGG TTGGGCCCGA TGCAC; Igκ: SEQ ID NO:12 - GATGAAGACA CTTGGTGCAG CCACAG; Igλ: SEQ ID NO:13: GGAACAGAGT GACACTGGGT GCAGCCTTGG GCTG). Recombinant plasmids identified previously as having the clonal V_{H} or V_{L} sequences are used as templates for a second round of PCR. Cycling conditions were as described *supra.*

Plasmid templates were combined with an IgG_{γ1}, IgM, Igλ, or Igκ constant region primer complementary to codon encoding amino acids 141-149, 115-123, 108-119, and 109-117 respectively and the appropriate leader/V region fusion primer. For example, for one patient, the primers used were SEQ ID NO:67 for V_{H3} and SEQ ID NO:68 for V_{κ3}(SEQ ID NO:67: CAGATCACTA GTTTTTATGG TCGTGTACAT TTCTTACATC TATGCGGAGA TGAAATTGGT GGAGTCTGGG; SEQ ID NO:68: CTGAGTAGGC CTGAGGCTAC AGCTCTCCCT GGGCGAAGTT GTGTTGACTC AGTCTCC). Cycling conditions were as described above.
a. Light Chain Variable Region Insertion into Expression Vector: A PCR derived V_{L} product and 2 µg of the corresponding pTRABacHuLC_{κ}HC_{γ1} or pTRABacHuLC_{λ}HC_{γ1} cassette vector digested with *Stu* I and *Dra* III. The 350 bp DNA fragment from the patient derived V_{L} region and the 8.4 kb fragment for the linear pTRABacHuLC_{κ}HC_{γ1} or pTRABacHuLC_{λ}HC_{γ1} vector were purified from agarose gel plugs with Qiaex II resin and eluted in 50 µl water. The DNA concentrations for both fragments were determined and then the fragments ligated using Rapid Ligation kit (Roche). The ligation products were used to transform competent XL1-Blue *E*. *coli* which were subsequently plated on a LB-carbenicillin agar plate for overnight growing at 37 °C. Miniprep colonies were prepared and the recombinant DNA plasmids were verified by restriction analysis and sequencing. The resulting vector designated pTRABac(NHL-V_{L})C_{κ}HC_{γ1} or pTRABac(NHL-V_{L})LC_{λ}HC_{γ1}.
b. Heavy Chain Variable Region Insertion into Expression Vector: A PCR derived V_{H} product and 2 µg of the pTRABac(NHL-V_{L})LC_{κ}HC_{γ1} or pTRABac(NHL-V_{L})LC_{λ}HC_{γ1} cassette vector were digested with *Spe* I and *Apa* I. The 350 bp DNA fragment from the patient derived V_{H} region and the 8.8 kb fragment for the linear pTRABac(NHL-V_{L})LC_{κ}HC_{γ1} or pTRABac(NHL-V_{L})LC_{λ}HC_{γ1} vector were purified from agarose gel plugs with Qiaex II resin and eluted in 50 µl water. The DNA concentrations for both fragments were determined and then the fragments ligated using Rapid Ligation kit (Roche). The ligation products were used to transform competent XL1-Blue *E*. *coli* which were subsequently plated on a LB-carbenicillin agar plate for overnight growing at 37 °C. Miniprep colonies were prepared and the recombinant DNA plasmids were verified by restriction analysis and sequencing. The resulting vector is designated pTRABar-(NHL-V_{L})LC_{κ}(NHL-V_{H})HC_{γ1}, pTRABac(NHL-V_{L})LC_{λ}(NHL-V_{H})HC_{γ1} and is assigned a reference number corresponding to a patient, *e.g*., FV8786-001.

| **TABLE 2.** | **Primer Sequences Used for Construction of pTRABacHuLC_{κ}HC_{γ1} and pTRABacHuLC_{λ}HC_{γ1} Baculovirus Transfer Vectors.** |
|---|---|
| | |

| PRIMER NAME | PRIMER SEQUENCE (5' 3') |
|---|---|
| | |
| 1. Melittin N-terminus (MelS/N and MelN/S) | ACTAGTGCAACGTTGACTAAGAATTTCATGCGGCCGC (SEQ ID NO:15) |
| | GCGGCCGCATGAAATTCTTAGTCAACGTTGCACTAGT (SEQ ID NO:16) |
| 2. Human Placental Alkaline Phosphatase N-terminus (APB/E and APE/B) | GCGGATCCATGGTGGACCCTGCATGCTGCTGCTGCT GCTGCTGCTAGGCCTggaattcc (SEQ ID NO:17) |
| | GGAATTCCAGGCCTAGCAGCAGCAGCAGCAGCAGCA TGCAGGGTCCCACCATGGATCCGC (SEQ ID NO:18) |
| 3. IgG_{γ1} Heavy Chain Constant: Upstream | TGTGACTAGTATGTATCGGCCCATCGGTCTTCCCCCT (SEQ ID NO:19) |
| Downstream | TTTCTAGACTATTATTTACCCGGAGACAGGGAGAG (SEQ ID NO:20) |
| 4. Kappa Light Chain Constant Upstream | CTAGGCCTATGTATCACCAAGTGTCTTCATCTTCCCGC CATCT (SEQ ID NO:21) |
| Downstream | CCCAAGCTTCTATTAACACTCTCCCCTGTTGAAGCT (SEQ ID NO:22) |

### 4. TRANSFECTION OF INSECT CELL LINES WITH VARIABLE REGION-CONTAINING EXPRESSION VECTORS AND PRODUCTION OF RECOMBINANT CHIMERIC PROTEINS:

**a. Insect Cell Growth:** Two established insect cell lines (Sf9 and High-5) were transfected with modified baculoviral vectors to produce recombinant chimeric V_{H}/immunoglobulin and/or V_{L}/immunoglobulin proteins. All insect cells were grown at 28 °C in ESF-921 Serum Free Insect Media (Expression Systems LLP) containing 50 µg/L gentamycin in disposable sterile vented shaker flasks (Coming), at 140-150 rpm, with no more than 50% liquid volume. Cells were passaged every 2 to 3 days. Frozen cells were thawed (Cryo-preservation media: 10% DMSO, 40% ESF-921 medium, 50% High-5 conditioned media) from a working cell bank for each lot of product or every six weeks to assure a continuous stock of exponentially growing cells that was not retractile to infection by baculovirus.
**b. S*f*9 Cell Transfection and Recombination Assay: The modified** baculovirus expression vectors containing genes for V_{H} and/or V_{L} regions and genes encoding immunoglobulin heavy and/or light chain constant regions were co-transfected into *Sf9* cells using the BacVector-3000 transfection kit (Invitrogen). Ten individual plaques are picked from agarose overlays. Virus from isolated plaques are used to infect T-25 flasks seeded with S*f*-9 cells at 50% confluency in 5 ml ESF-921 media. Clonal viral isolates amplified in T-25 flasks are tested by PCR, using two primers (SEQ ID NO:36 - TTTACTGTTT TCGTAACAGT TTTG) and (SEQ ID NO:37 - GGTCGTTAAC AATGGGGAAG CTG) to assure clonality of the isolated plaques and that there was no wild type virus contamination. In general, 200 ng recombinant transfer vector plasmid was co-transfected with triple-cut Bac-Vector-3000 as described in the Bac Vector manual (Novagen) using the Eufectin lipid reagent supplied. This transfection mixture was subjected to serially 5-fold dilutions. One hundred microliter aliquots were plated in 60 mm tissue culture dishes containing 2.5 x 10⁶ adherent Sf9 cells. After 1 hour, cells were overlaid with 4 ml of a 1% agarose solution in ESF-921 culture medium. Ten individual clones were picked from the transfected cells grown in agarose overlays after staining for live cells using Neutral Red (Sigma, St. Louis, MO) at t=144 hours post transfection. Virus was eluted from plaque plugs overnight in 1 ml ESF-921 media. T-25 flasks were seeded with Sf-9 cells at 50% confluency in 5 ml ESF-921 media, and infected with 0.5 ml of eluted clonal virus. Ninety-six hours post infection, 0.5 ml media was removed from T-25 flasks; the cells were removed by centrifugation and the supernatant was assayed for immunoglobulin activity by dot blotting on nitrocellulose. The absence of wild type virus was also tested by PCR as follows.
   Infectious supernatant (10 µl) containing recombinant baculovirus was added to 90 µl of lysis buffer containing 10 mM Tris pH 8.3, 50 mM KCl, 0.1 mg/ml gelatin, 0.45% Nonidet P-40, and 0.45% Tween-20, containing 6 µg Proteinase-K. The mixture was heated for 1 hour at 60 °C and the Proteinase-K was denatured by incubation at 95 °C for 10 min. Twenty five µl of the heated mixture was removed to a fresh PCR tube after cooling, and another 25 µl of the mixture containing 10 mM Tris pH 8.3, 50 mM KCl, 0.1 mg/ml gelatin, 0.45% NP-40, 0.45% Tween-20, 400 µM each dNTP, 5 mM MgCl₂, 50 pM each PCR primer (final), and 2.5 U Taq polymerase (Roche) was added. The viral DNA was amplified for 40 cycles at: 92 °C for 1 min., followed by 58 °C for 1 min. and 72 °C for 1 min. The recombinant baculovirus primers PH forward (SEQ ID NO:36) and PH reverse (SEQ ID NO:37) were used to amplify the polyhedron locus expressing the light chain gene. PCR products were analyzed following electrophoresis through an agarose gel. Recombinant baculovirus would amplify a 1300 bp fragment, while wild type baculovirus would produce a ~ 800 bp fragment with these primer sets. Recombinant virus contaminated with wild type virus would amplify both fragment sizes.
**c. Preparation of High Titer Viral Stocks in *S*f*9* Insect Cells: Two ml** from a T-25 primary culture was transferred to a T-75 flask containing *Sf9* cells at 50% confluency in 10 ml ESF-921 media, and cells were grown for 120 hours at 28 °C. Five ml of secondary T-75 cultures was transferred to a 150 ml shaker flask containing 50 ml of *Sf9* cells at 2 x 10⁶ cell/lml, and cells were grown for 120 hours at 28 °C. 25 ml was transferred from the 150 ml shaker flask into 500 ml of Sf-9 cells at 2 x 10⁶ cells/ml in a one liter shaker flask, and was grown at 28 °C. When the cultures reached 20%, viable cells as determined by trypan blue staining (approximately 120 to 144 hours post infection), the viral culture was harvested by centrifugation at 3000 x g, distributed into 50 ml sterile tubes, and half of the tubes were stored at 4 °C with the rest at -80 °C. This harvested 500 ml high titer (>1 x 108 pfu/ml) viral stock was then used to infect High-5 insect cells for immunoglobulin production. Viral liters (pfu/ml) were determined using a Baculovirus Rapid Titer Kit (Clontech, Palo Alto, CA).
**d. Production of Id in High-5 Insect Cells:** High-5 insect cells (BTI-TN-5B1-4) secreted higher levels (2-20 X) of recombinant immunoglobulin compared to *Sf9* cells, and were chosen for chimeric protein production. Early log phase High-5 cells (1.0-2.0 x 10⁶ cells/ml) were seeded in 1 liter disposable culture flasks with vented closures at 5 x 10⁵ cells/ml in ESF921 Media (Expression Systems LLP). The flasks were shaken at 140-150 rpm at 28 °C, and the volume of media in the flasks was adjusted over time to no greater than 500 ml. When the cell densities reached 1.5 - 2.5 cell/ml in 500 ml media, the flasks were infected with high titer recombinant baculovirus stock at a multiplicity of infection (MOI) approximating 0.5:1 (pfu:cells). The flasks were then shaken at 140-150 rpm at 28 °C; the culture was harvested 96 hours post-infection.

### 5. PURIFICATION OF THE CHIMERIC PROTEIN COMPRISING A V_{H}-IMMUNOGLOBULIN AND A V_{L}-IMMUNOGLOBULIN:

Cells and debris were removed by centrifugation for 60 min. at approximately 5,000 x g, followed by filtration through a 0.2µ PES sterile filter unit. Chimeric proteins were purified from cleared tissue culture media by affinity chromatography with a Protein-A High-Trap cartridge (Amersham Pharmacia, Piscataway, NJ), followed by ion-exchange chromatography utilizing FPLC technology (Amersham Pharmacia). The purified chimeric proteins were size separated and buffer exchanged into PBS by FPLC chromatography. All reagents used for protein purification were of USP biotechnology grade (GenAr, Mallinckrot Baker, Parris, KY) and endotoxin tested by the manufacturer. Sterile USP grade water was used to make all buffers and other solutions. Buffers and other solutions were prepared in a biological safety cabinet, and filter sterilized through 0.2 µm PES filter units.
**a. Protein A Sepharose Affinity Purification of the Chimeric Proteins:** Tissue culture medium was removed from growing culture flasks and spun for 60 min. at 5,000 x g to sediment cells and debris. The supernatant was sterilized by filtration using a 0.2µ PES filter unit. Tris buffer (1M, pH 7.4) was added to the filtered medium containing V_{H} and/or V_{L}-immunoglobulin chimeric proteins to a final concentration of 20 mM. The buffered tissue culture supernatant was loaded onto a 5 ml HighTrap recombinant Protein A Sepharose affinity cartridge at a flow rate of 1 to 5 ml/min with a P1 peristaltic pump (Amersham Pharmacia) collecting the flow-through in a clean flask. The column was washed with 25 ml PBS (pH 7.4) at 5 ml/min. The direction of the flow was reversed and the column was washed with an additional 25 ml PBS. The column was eluted in reverse at 1 ml/min with 0.05 M citric acid (pH 3.5) collecting 1 ml fractions. Other protein columns including but not limited to protein G, protein L, or any proteins that are able to bind to an immunoglobulin binding domain could be used in the same manner.
**b. Ion Exchange Chromatography:** A 5 ml High Trap SP Sepharose cation exchange cartridge was equilibrated with 50 ml of 25 mM citric acid (pH 3.5) and 20 mM NaCl. The Protein A eluted V_{H} and/or V_{L}-IgG chimeric proteins were loaded directly onto the equilibrated High Trap SP Sepharose column using a peristaltic pump at a flow rate of 1 ml/min. The column was washed with 25 ml 50 mM citric acid (pH 3.5) and 20 mM NaCl (Buffer A) at 2 ml/min. The column was eluted with a linear gradient (0% Buffer B to 100% Buffer B) to collect 1 ml fractions at 1 ml/min. (Buffer B =100 mM Na carbonate (pH 10.0) and 1M NaCl). The ion exchange eluted fractions containing V_{H} and/or V_{L}-IgG chimeric proteins were analyzed spectrophotometrically by their OD₂₈₀. The peak fractions were pooled.
**c. Size Exclusion Chromatography:** The pooled Ig fraction from SP ion-exchange was then loaded onto a Hi Prep Sephacryl 26/60 S200 Hi Resolution column (Pharmacia) that had been equilibrated in 5 column volumes of PBS (pH7.2) following a pre-wash in 100 ml sterile water. The chimeric Ig proteins were eluted in PBS at a flow rate of 0.5 ml/min and collected in 1 ml fractions. The major Ig peak was apooled a sterile filtered through a 0.2µ filter.

### 6. IDIOTYPIC PROTEIN AND KEYHOLE LIMPET HEMOCYANIN (KLH) CONJUGATION.

Once purified, the idiotypic protein was conjugated to GMP grade KLH (VACMUNE, Biosyn Corporation) via glutaraldehyde crosslinking. At least 5 mg of purified, sterile idiotypic protein as described, *supra,* was combined with an equal weight ofKLH in a sterile 15 ml conical tube and the final volume was adjusted to 9 ml in PBS. One ml of 1% glutaraldehyde (25% Grade 1 aqueous solution, Sigma) was added dropwise to a final concentration of 0.1%. The tube was then slowly rocked for 4 hours at room temperature. The conjugate was dialyzed in sterile DispoDialyzers (Spectrum Labs) against 2 liters sterile PBS, with three buffer changes over at least 24 hours in a biological safety hood. The final IgG/KLH conjugate in PBS is aseptically removed from the dialysis chambers and transferred into a sterile tube, mixed, then aliquoted in vials. Each vial of final product was labeled with the lot number, patient identifier, vial number and date vialed. Ten percent of the final vialed lot was tested for sterility and a vial was tested for the presence of endotoxin. One vial was retained for archival purposes.

### 7. PRODUCT TESTS

**a. DNA Sequence of Baculovirus Containing Production Lot** Supernantant: A 1 ml aliquot of sample of infected insect cell production culture supernatant was harvested and cleared of cellular debris by spinning at 3000 rpm for 5 min in a desktop centrifuge. At least 0.1 ml of this cleared supernatant containing baculovirus particles was combined at a volume ratio of 1 to 9 with lysis buffer (10mM Tris pH 8.3, 50 mM KCl, 0.1 mg/ml gelatin, 0.45% Nonidet P-40, and 0.45% Tween-20), subjected to proteolysis with proteinase K (final concentration 60 µg/ml) for 1 h at 60 °C, followed by denaturation for 15 min at 95 °C. Twenty-five µl of this lysate was then combined with an additional 25 µl of the above lysis buffer containing 400 µM each dNTP, 5 mM MgCl₂, 25 pmol forward and reverse oligonucleotide primers (see Table 3; SEQ ID NO:34 and SEQ ID NO:31 for V_{H} Identification and SEQ ID NO:35 and SEQ ID NO:36 for V_{L} identification, respectively), and 2.5 U Taq polymerase (Roche). Cycling conditions for the PCR of V_{L} are: initial denaturation for 2 min at 92 °C, followed by 40 cycles of 1 min each at 92 °C, 58 °C, and 72 °C, with a final extension of 7 min at 72 °C. For the PCR of V_{H}, cycling conditions are the same except that the annealing temperature is 64 °C. PCR products were assessed for expected size and quantity by agarose gel electrophoresis. Subsequently, two or more nested primers were used to directly sequence the PCR products. (See Table 3; SEQ ID NO:30 and SEQ ID NO:34 for V_{H} identification, SEQ ID NO:28 and SEQ ID NO:35 for V_{K} identification, and SEQ ID NO:88 and SEQ ID NO:35 for Vλ identification, respectively.) The complete V_{H} and V_{L} nucleotide sequences was determined using the the OpenGene Automated DNA Sequencing System (Visible Genetics) and sequencing analysis software, as described above and compared with the V-gene sequences of the pTRABac(NHL-FV-8786-XXX) vector corresponding to that patient's idiotype.
**b. Superose 6 Gel Filtration Chromatography:** Gel filtration chromatography of the purified Id was performed to assess protein purity. Gel filtration chromatography was performed using a Superose 6 HR 10/30 FPLC column (Amersham Pharmacia) with PBS as the liquid phase. Peak integration was performed on the largest 20 peaks by the FPLC software using the following criteria to reject a peak from being included in area evaluation: height less than 0.01 Au; width less than 0.05 ml; area less than 0.01 Au/ml. Fractions of each column run were collected and assayed for human immunoglobulin specific activity by capture ELISA, and compared to the OD₂₈₀ chromatogram.
**c. Immnnoglobulin Assay; Anti Human IgG ELISA:** Microtiter plate wells were coated with 100µl of a 3 µg/ml dilution of Goat anti-Human IgG heavy chain specific antibody (Roche) in carbonate buffer overnight at 4 °C, and washed 2 times with 100µl TBS (50mM Tris, 150mM NaCl, pH 7.5). Wells were blocked with of 200µl TBSB (TBS + 1% BSA) for 1 hour at 22 °C.
   Each chromatogram fraction corresponding to human peak in TBSB was tested. One hundred µl of diluted sample was added in 2-fold serial dilutions to wells in replicates, and incubated 1 hour at 22 °C. The assay was repeated with purified Human IgG1/κ or IgG1/λ standards (Sigma, St. Louis, MO). The wells were washed 4 times with 200µl TBST (TBS + 0.1% Tween 20). The detection antibody was diluted (Goat-anti-Human κ or λ-HRP (Fischer, Pittsburgh, PA)1:2000 in TBSB, 100µl was added to wells, and incubated for 1 hour at 22 °C. The wells were washed 6 times with 200µl TBST. One hundred µl of substrate (TMB 1 component, KPL Inc., Gaithersburg, MD) was added to wells, developed 30 min. and assayed at OD₆₂₀.
**d. Idiotypic Protein Release Criteria:** (1) The DNA sequence of idiotype-variable genes in baculovirus from production supernantant must be identical to the DNA sequence in the production vector. (2) The idiotypic protein concentration was greater than 0.5 mg/ml based on OD₂₈₀. (3) The major peak area was greater than 90% of area in evaluated peaks on Superose 6 analytical chromatography. (4) The major chromatographic peak corresponds to the human IgGκ (or λ) ELISA activity peak.

The final vaccine product, Id-KLH, was tested for endotoxin levels by a kinetic turbidity microplate assay or a Limulus Amoebocyte Lysate (LAL) assay and had a level below 350 endotoxin units (EU) per ml. Ten percent of the lot was tested for sterility on a 14-day test and tests negative or was discarded.

Table 3 shows a summary of primer sequences used for establishing final product identity.

| **TABLE 3. Primer Sequences Used for Establishing Final Product Identity.** | |
|---|---|
| | |

| PRIMER NAME | PRIMER SEQUENCE (5' 3') |
|---|---|
| | |
| 1. Human Placental Alkaline Phosphatase Internal | AAATGATAACCATCTCGC (SEQ ID NO:25) |
| 2. Human Placental Alkaline Phosphatase External | TTTACTGTTTTCGTAACAGTTTTG (SEQ ID NO:26) |
| 3. Kappa Light Chain Constant Antisense | TTGGAGGGCGTTATCCACCTTC (SEQ ID NO:27) |
| 4. Kappa Light Chain Constant Downstream Internal | CTGTAAATCAACAACGCACAG (SEQ ID NO:28) |
| 5. Kappa Light Chain Constant Downstream External | CAACAACGCACAGAATCTAG (SEQ ID NO:29) |
| 6. Melittin Internal | GGGACCTTTAATTCAACCCAACAC (SEQ ID NO:30) |
| 7. Melittin External | AAACGCGTTGGAGTCTTGTGTGC (SEQ ID NO:31) |
| 8. IgG_{γ1} Heavy Chain Constant Downstream Internal | GGAAGTAGTCCTTGACCAGGCAG (SEQ ID NO:32) |
| 9. IgG_{γ1} Heavy Chain Constant | CTGAGTTCCACGACACCGTCAC |
| Downstream Middle | (SEQ ID NO:33) |
| 10. IgG_{γ1} Heavy Chain Constant Downstream External | TAGAGTCCTGAGGACTGTAGGAC (SEQ ID NO:34) |
| 11. Kappa & Lambda Downstream: | 5'-GGTCGTTAACAATGGGGAAGCTG-3' (SEQ ID NO:35) |
| 12. PH forward | 5'-TTTACTGTTTTCGTAACAGTTTTG-3' (SEQ ID NO:36) |
| 13. PH reverse | 5'-GGTCGTTAACAATGGGGAAGCTG-3' (SEQ ID NO:37) |
| 14. Lambda Constant Internal | 5'-GAAGTCACTTATGAGACACACCAG-3' (SEQ ID NO:88) |

### 8. USE OF CHIMERIC PROTEIN OF THE INVENTION FOR TREATMENT OF NON-HODGKIN'S B-CELL LYMPHOMA:

V_{H} and V_{L} regions were obtained from a patient with Non-Hodgkin's B-Cell Lymphoma. Using the 5' RACE method described *supra,* genes encoding these regions were cloned and inserted into the expression vector and expressed by the methods of the instant invention. Table 5 contains the DNA sequences of the Vh and VI regions used for the expression vector. The Apa I and Dra III sites used for cloning are indicated by underlining.

**Table 5: Variable region sequences obtained from a patient.**

| |
|---|
| VH A / 07 |
| GACATGTTGTTGGTGGAATCGGGGGAGGCCTGGTCCAGCCGGGGAGTCCCTGAGACT CTCCTGTGTGGCCTCTAGATTCACCTTTAGAACGTTTTGGATGACCTGGGTCCGCCAAC TTCCAGGGAAGGGGCTGGAGTGGGTGGCCAATATAAATCAAGATGGCAGTCAGACGTAT CATGCGGACTCTGTAAAGGCCGATTTACCATCTCCAGAGACAACGGCAGGAACTCCCT ATTTTTACAAATGACAAGTCTGAGAGTCGCGGACACGGCTATATATTACTGTGCGACTA ATGAAACGTCCAGTGGCCTGGACTGCTGGGGCCAAGGAACCCTGGTCACTGTCTCCTCA GCTTCCACCAAGGGCCC SEQ ID NO:86 |
| VKA / L6 |
| GAAATCGTGTTGACACAGTCTCCAGCCACCCTGTCTTCGTCTCCAGGAGACAGAGTCGC CCTCTCCTGCAGGGCCAGTCAGAGTGTAAGAAGTTACTTAAGTTGGTATCAACAGAAGG CTGGCCAGGCTCCCAGGCTCCTCATCCATAATGCATCCAGTAGGGCCACTGGCATCCCG CCCAGATTCAGTGGCAGTGGGTCTGGGACAGACTTCACTCTCACCATCAGTCGCCTAGA GACTGAAGATGCTGCAGTTTATTACTGTCAGCAACTTTATTTCTGGCCTCCGATATTAT TTTTCGGCCCTGGGACCAAAGTGAATATCACACGAACTGTGGCTGCACCAAGTG SEQ ID NO:87 |

The isolated recombinant chimeric immunoglobulin protein produced for this patient from the genetic information detailed above was conjugated to KLH and administered with GM-CSF five times over a six-month period as described *supra.* A CT scan was performed on the neck and pelvis areas of the patient prior to administration of the therapy and 9 months later. A comparison of the sum of the diameters of 6 tumor masses revealed a 60% reduction nine months following therapy initiation. (Note that these figures are not adjusted to accommodate the size of the lymph node prior to diagnosis of the disease (See, Cheson et al., J. Clin. Oncol., 17(4):1244, 1999.)

**Table 6: Reduction in size of lymph nodes following treatment.**

| | PRIOR TO TXT. (Product of diameters; cm²) | 9 MONTHS POST TXT. (Product of diameters; cm²) |
|---|---|---|
| | | |
| LYMPH NODE 1 | 6.16 | 2.8 |
| LYMPH NODE 2 | 5.0 | 1.6 |
| LYMPH NODE 3 | 3.3 | 1.17 |
| LYMPH NODE 4 | 3.78 | 1.44 |
| LYMPH NODE 5 | 1.92 | 1.0 |
| LYMPH NODE 6 | 1.08 | 0.80 |
| SUM OF DIAMETERS | 21.24 | 8.81 |

## Claims

1. Use of a first chimeric protein and a second chimeric protein for the manufacture of a composition for therapy of a B cell lymphoma in a human patient,
wherein said first chimeric protein comprises at least a portion of a V_{H} region and at least a portion of an IgM immunoglobulin constant region; and
wherein said second chimeric protein comprises at least a portion of a V_{L} region and at least a portion of a second immunoglobulin constant region;
wherein said manufacture comprises producing said chimeric proteins by a method comprising:
(a) isolating a gene encoding said portion of said V_{H} region from B cells associated with said patient's B cell lymphoma;
(b) inserting said gene encoding said portion of said V_{H} region and a gene encoding said portion of said IgM immunoglobulin constant region into an expression vector to allow the expression of said first chimeric protein;
(c) isolating a gene encoding said portion of said V_{L} region from B cells associated with said patient's B cell lymphoma;
(d) inserting said gene encoding said portion of said V_{L} region and a gene encoding said portion of said second immunoglobulin constant region into an expression vector to allow the expression of said second chimeric protein; and
(e) producing said chimeric proteins by introducing expression vector into an insect cell line;
wherein the composition further comprises a carrier protein conjugated to said chimeric protein.

2. The use according to claim 1, wherein said V_{H} or V_{L} region is an entire variable region.

3. The use according to claim 1 or 2, wherein said carrier protein is keyhole-limpet hemocyanin (KLH).

4. The use according to any one of the preceding claims, wherein said composition is for co-administration with a cytokine or chemokine.

5. The use according to claim 4, wherein said cytokine is granulocyte-macrophage-colony stimulating factor (GM-CSF).

6. The use according to claim 4, wherein said chemokine is monocyte chemotactic protein 3 (MCP 3).

7. The use according to any one of the preceding claims, wherein the composition is for administration by injection, inhalation, oral or transdermal delivery.

8. The use according to any one of the preceding claims, wherein said B cell lymphoma is a refractory low grade lymphoma or follicular B cell lymphoma.

9. The use according to any one of claims 1 to 7, wherein said B cell lymphoma is non-Hodgkins lymphoma.

10. The use according to any one of the preceding claims, wherein said expression vector is a baculovirus expression vector.

11. The use according to claim 10, wherein said baculovirus expression vector comprises a honey bee melittin secretory signal sequence and a human placental alkaline phosphatase secretory signal sequence.

12. The use according to claim 10, wherein said baculovirus expression vector further comprises a baculovirus AcNPV p10 promoter and AcNPV polyhedrin promoter, wherein said p10 promoter controls a honey bee melittin secretory signal sequence, and wherein said polyhedrin promoter controls a human placental alkaline phosphatase secretory signal sequence.

13. The use according to claim 12, wherein said gene encoding a chimeric protein comprising a V_{H} region and an IgM immunoglobulin constant region is controlled by said p10 promoter in said baculovirus expression vector, and said gene encoding a chimeric protein comprising a V_{L} region and a second immunoglobulin constant region is controlled by said polyhedrin promoter in said baculovirus expression vector.

14. The use according to claim 12, wherein said gene encoding said V_{H} or V_{L} region and said immunoglobulin constant region is controlled by either said p10 promoter or polyhedrin promoter in said baculovirus expression vector.

15. The use according to any one of the preceding claims, wherein said insect cell line is a *Trichoplusia ni* (Hi-5) or a *Spodoptera frugiperda* (sf9) cell line.

16. The use according to any one of the preceding claims, wherein said chimeric proteins are analyzed for expression by ELISA.

17. The use according to any one of the preceding claims, wherein said chimeric proteins are isolated using a protein selected from the group consisting of protein A, protein G, protein L and other proteins being able to bind to an immunoglobulin binding domain.

18. The use according to claim 17, wherein said other protein able to bind an immunoglobulin binding domain is an anti-immunoglobulin antibody.

## Patentansprüche

1. Verwendung eines ersten chimären Proteins und eines zweiten chimären Proteins für die Herstellung einer Zusammensetzung zur Therapie eines B-Zell-Lymphoms bei einem menschlichen Patienten,
wobei das erste chimäre Protein wenigstens einen Teil einer V_{H}-Region und wenigstens einen Teil einer konstanten Region von IgM-Immunglobulin umfasst und
wobei das zweite chimäre Protein wenigstens einen Teil einer V_{L}-Region und wenigstens einen Teil einer zweiten konstanten Immunglobulin-Region umfasst;
wobei die Herstellung Produzieren der chimären Proteine durch ein Verfahren, umfassend:
(a) Isolieren eines Gens, das den Teil der V_{H}-Region kodiert, aus B-Zellen, die mit dem B-Zell-Lymphom des Patienten assoziiert sind;
(b) Inserieren des Gens, das den Teil der V_{H}-Region kodiert, und eines Gens, das den Teil der konstanten Region von IgM-Immunglobulin kodiert, in einen Expressionsvektor, um die Expression des ersten chimären Proteins zu ermöglichen;
(c) Isolieren eines Gens, das den Teil der V_{L}-Region kodiert, aus B-Zellen, die mit dem B-Zell-Lymphom des Patienten assoziiert sind;
(d) Inserieren des Gens, das den Teil der V_{L}-Region kodiert, und eines Gens, das den Teil der zweiten konstanten Immunglobulin-Region kodiert, in einen Expressionsvektor, um die Expression des zweiten chimären Proteins zu ermöglichen, und
(e) Produzieren der chimären Proteine durch Einführen des Expressionsvektors in eine Insektenzelllinie, umfasst;
wobei die Zusammensetzung außerdem ein Trägerprotein umfasst, das an das chimäre Protein konjugiert ist.

2. Verwendung gemäß Anspruch 1, wobei die V_{H}- oder V_{L}-Region eine ganze variable Region ist.

3. Verwendung gemäß Anspruch 1 oder 2, wobei das Trägerprotein Napfschneckenhämocyanin (keyhole-limpet hemocyanin, KLH) ist.

4. Verwendung gemäß einem der vorangehenden Ansprüche, wobei die Zusammensetzung für eine Co-Verabreichung mit einem Cytokin oder Chemokin ist.

5. Verwendung gemäß Anspruch 4, wobei das Cytokin Granulozyten-Macrophagen-Kolonie-stimulierender Faktor (GM-CSF) ist.

6. Verwendung gemäß Anspruch 4, wobei das Cytokin Monozytenchemotaktisches Protein 3 (MCP 3) ist.

7. Verwendung gemäß einem der vorangehenden Ansprüche, wobei die Zusammensetzung zur Verabreichung durch Injektion, Inhalation, orale oder transdermale Abgabe ist.

8. Verwendung gemäß einem der vorangehenden Ansprüche, wobei das B-Zell-Lymphom ein refraktäres geringgradiges Lymphom oder follikuläres B-Zell-Lymphom ist.

9. Verwendung gemäß einem der Ansprüche 1 bis 7, wobei das B-Zell-Lymphom Nicht-Hodgkin-Lymphom ist.

10. Verwendung gemäß einem der vorangehenden Ansprüche, wobei der Expressionsvektor ein Baculovirus-Expressionsvektor ist.

11. Verwendung gemäß Anspruch 10, wobei der Baculovirus-Expressionsvektor eine sekretorische Signalsequenz von Honigbienen-Melittin und eine sekretorische Signalsequenz von humaner plazentaler alkalischer Phosphatase umfasst.

12. Verwendung gemäß Anspruch 10, wobei der Baculovirus-Espressionsvektor außerdem einen Baculovirus-AcNPV-p10-Promotor und -AcNPV-Polyhedrin-Promotor umfasst, wobei der p10-Promotor eine sekretorische Signalsequenz von Honigbienen-Melittin reguliert und wobei der Polyhedrin-Promotor eine sekretorische Signalasequenz von humaner plazentaler alkalischer Phosphatase reguliert.

13. Verwendung gemäß Anspruch 12, wobei das Gen, das ein chimäres Protein kodiert, das eine V_{H}-Region und eine konstante Region von IgM-Immunglobulin umfasst, durch den p10-Promotor in dem Baculovirus-Expressionsvektor reguliert wird und das Gen, das ein chimäres Protein kodiert, das eine V_{L}-Region und eine zweite konstante Region von Immunglobulin umfasst, durch den Polyhedrin-Promotor in dem Baculovirus-Expressionsvektor reguliert wird.

14. Verwendung gemäß Anspruch 12, wobei das Gen codiert die V_{H}- oder V_{L}-Region oder die konstante Immunglobulin-Region, entweder durch den p10-Promotor oder Polyhedrin-Promotor in dem Baculovirus-Expressionsvektor reguliert wird.

15. Verwendung gemäß einem der vorangehenden Ansprüche, wobei die Insektenzelllinie eine Trichoplusia-ni(Hi-5)- oder eine Spodoptera-frugiperda(sf9)-Zellline ist.

16. Verwendung gemäß einem der vorangehenden Ansprüche, wobei die chimären Proteine durch ELISA auf Expression analysiert werden.

17. Verwendung gemäß einem der vorangehenden Ansprüche, wobei die chimären Proteine unter Verwendung eines Proteins, ausgewählt aus der Gruppe, bestehend aus Protein A, Protein G, Protein L und anderen Proteinen, die fähig sind, an eine Immunglobulinbindungsdomäne zu binden, isoliert werden.

18. Verwendung gemäß Anspruch 17, wobei das andere Protein, das fähig ist, an eine Immunglobulinbindungsdomäne zu binden, ein Anti-Immunglobulin-Antikörper ist.

## Revendications

1. Utilisation d'une première protéine chimérique et d'une deuxième protéine chimérique pour la fabrication d'une composition destinée à la thérapie d'un lymphome à lymphocytes B chez un patient humain, étant entendu que :
ladite première protéine chimérique comprend au moins une partie d'une région V_{H} et au moins une partie d'une région constante d'immunoglobuline IgM,
ladite deuxième protéine chimérique comprend au moins une partie d'une région V_{L} et au moins une partie d'une deuxième région constante d'immunoglobuline,
et ladite fabrication comprend le fait de produire lesdites protéines chimériques par un procédé comprenant les étapes suivantes :
a) isoler un gène codant ladite partie de ladite région V_{H}, à partir de lymphocytes B associés au lymphome à lymphocytes B dudit patient ;
b) insérer ce gène codant ladite partie de ladite région V_{H} et un gène codant ladite partie de ladite région constante d'immunoglobuline IgM dans un vecteur d'expression, pour permettre l'expression de ladite première protéine chimérique ;
c) isoler un gène codant ladite partie de ladite région V_{L}, à partir de lymphocytes B associés au lymphome à lymphocytes B dudit patient ;
d) insérer ce gène codant ladite partie de ladite région V_{L} et un gène codant ladite partie de ladite deuxième région constante d'immunoglobuline dans un vecteur d'expression, pour permettre l'expression de ladite deuxième protéine chimérique ;
e) et produire lesdites protéines chimériques, par introduction de vecteur d'expression dans une lignée de cellules d'insecte ;
étant entendu que la composition comprend en outre une protéine vectrice conjuguée à ladite protéine chimérique.

2. Utilisation conforme à la revendication 1, pour laquelle ladite région V_{H} ou V_{L} est une région variable entière.

3. Utilisation conforme à la revendication 1 ou 2, pour laquelle ladite protéine vectrice est de l'hémocyanine de fissurelle (KLH).

4. Utilisation conforme à l'une des revendications précédentes, ladite composition étant destinée à être administrée conjointement avec une cytokine ou une chimiokine.

5. Utilisation conforme à la revendication 4, pour laquelle ladite cytokine est du facteur GM-CSF (facteur de stimulation des colonies de granulocytes et macrophages).

6. Utilisation conforme à la revendication 4, pour laquelle ladite chimiokine est de la protéine MCP 3 (protéine chimiotactique monocytaire 3).

7. Utilisation conforme à l'une des revendications précédentes, la composition étant destinée à être administrée, par injection, par inhalation, par voie orale ou par voie transdermique.

8. Utilisation conforme à l'une des revendications précédentes, pour laquelle ledit lymphome à lymphocytes B est un lymphome de bas grade réfractaire ou un lymphome à lymphocytes B folliculaire.

9. Utilisation conforme à l'une des revendications 1 à 7, pour laquelle ledit lymphome à lymphocytes B est un lymphome non-hodgkinien.

10. Utilisation conforme à l'une des revendications précédentes, pour laquelle ledit vecteur d'expression est un vecteur d'expression baculovirus.

11. Utilisation conforme à la revendication 10, pour laquelle ledit vecteur d'expression baculovirus comprend une séquence signal de sécrétion de mélittine d'abeille et une séquence signal de sécrétion de phosphatase alcaline placentaire humaine.

12. Utilisation conforme à la revendication 10, pour laquelle ledit vecteur d'expression baculovirus comprend en outre un promoteur p10 de baculovirus AcNPV et un promoteur polyhédrine d'AcNPV, étant entendu que ledit promoteur p10 commande une séquence signal de sécrétion de mélittine d'abeille et que ledit promoteur polyhédrine commande une séquence signal de sécrétion de phosphatase alcaline placentaire humaine.

13. Utilisation conforme à la revendication 12, pour laquelle ledit gène qui code une protéine chimérique comprenant une région V_{H} et une région constante d'immunoglobuline IgM est commandé par ledit promoteur p10 dans ledit vecteur d'expression baculovirus, et ledit gène qui code une protéine chimérique comprenant une région V_{L} et une deuxième région constante d'immunoglobuline est commandé par ledit promoteur polyhédrine dans ledit vecteur d'expression baculovirus.

14. Utilisation conforme à la revendication 12, pour laquelle ledit gène codant ladite région V_{H} ou V_{L} et ladite région constante d'immunoglobuline est commandé soit par ledit promoteur p10, soit par ledit promoteur polyhédrine, dans ledit vecteur d'expression baculovirus.

15. Utilisation conforme à l'une des revendications précédentes, pour laquelle ladite lignée de cellules d'insecte est une lignée de cellules de Trichoplusia ni (Hi-5) ou de Spodoptera frugiperda (sf9).

16. Utilisation conforme à l'une des revendications précédentes, pour laquelle on analyse l'expression desdites protéines chimériques par la méthode ELISA.

17. Utilisation conforme à l'une des revendications précédentes, pour laquelle on isole lesdites protéines chimériques à l'aide d'une protéine choisie dans l'ensemble formé par les protéine A, protéine G, protéine L et autres protéines capables de se lier à un domaine de liaison d'immunoglobuline.

18. Utilisation conforme à la revendication 17, pour laquelle ladite autre protéine capable de se lier à un domaine de liaison d'immunoglobuline est un anticorps anti-immunoglobuline.
